(19) Europäisches Patentamt European Patent Office Office européen des brevets

(11) **EP 1 276 418 B1**

(12) **EUROPÄISCHE PATENTSCHRIFT**

(45) Veröffentlichungstag und Bekanntmachung des Hinweises auf die Patenterteilung:
**22.07.2009 Patentblatt 2009/30**

(21) Anmeldenummer: **01929556.7**

(22) Anmeldetag: **12.04.2001**

(51) Int Cl.:
**_A61B 7/00_** _(2006.01)_

(86) Internationale Anmeldenummer:
**PCT/EP2001/004240**

(87) Internationale Veröffentlichungsnummer:
**WO 2001/078645 (25.10.2001 Gazette 2001/43)**

(54) **VORRICHTUNG ZUR ERMITTLUNG DES ANATOMISCHEN ZUSTANDS EINES MENSCHEN ODER EINES TIERES**

DEVICE FOR DETERMINING THE ANATOMICAL CONDITION OF A HUMAN BEING OR AN ANIMAL

DISPOSITIF DE DETERMINATION DE L'ETAT ANATOMIQUE D'UN HOMME OU D'UN ANIMAL

(84) Benannte Vertragsstaaten:
**AT BE CH CY DE DK ES FI FR GB GR IE IT LI LU MC NL PT SE TR**

(30) Priorität: **14.04.2000 DE 10018654**

(43) Veröffentlichungstag der Anmeldung:
**22.01.2003 Patentblatt 2003/04**

(73) Patentinhaber: **Czernicki, Jacek**
**81369 München (DE)**

(72) Erfinder: **Czernicki, Jacek**
**81369 München (DE)**

(74) Vertreter: **Manitz, Finsterwald & Partner GbR**
**Martin-Greif-Strasse 1**
**80336 München (DE)**

(56) Entgegenhaltungen:
DE-A- 4 030 448    US-A- 3 847 141
US-A- 4 437 473    US-A- 4 823 807
US-A- 5 213 555    US-A- 5 806 520
US-A- 5 931 763    US-A- 6 024 711

- BUKHMAN E V ET AL: "SPECTRAL ANALYSIS OF ACOUSTIC VIBRATIONS ON THE SURFACE OF THE HUMAN BODY" ACOUSTICAL PHYSICS, AMERICAN INSTITUTE OF PHYSICS. NEW YORK, US, Bd. 41, Nr. 1, 1995, Seiten 41-48, XP000500512 ISSN: 1063-7710
- SANJEEV TAVATHIA ET AL: "ANALYSIS OF KNEE VIBRATION SIGNALS USING LINEAR PREDICTION" IEEE TRANSACTIONS ON BIOMEDICAL ENGINEERING, IEEE INC. NEW YORK, US, Bd. 39, Nr. 9, 1. September 1992 (1992-09-01), Seiten 959-970, XP000322974 ISSN: 0018-9294

**Beschreibung**

Die vorliegende Erfindung befaßt sich mit einer Vorrichtung zur Ermittlung von Informationen bezüglich des anatomischen Zustands eines Bestandteils des Körpers eines Menschen oder eines Tieres, beispielsweise eines Rennpferdes oder eines Hundes, bestehend aus:

- einer Tonaufnahmevorrichtung zur Aufnahme von Geräuschen, die durch die Bewegung eines dem Bestandteil zugeordneten Gelenks entstehen und zur Erzeugung von entsprechenden Signalen,
- einer Analysevorrichtung zur Auswertung der Signale in Form einer Signalanalyse und
- einer Anzeigevorrichtung zur Darstellung des Ergebnisses der Signalanalyse, wobei die Analysevorrichtung durch einen Rechner (34) und die Anzeigevorrichtung durch einen dem Rechner zugeordneten Bildschirm gebildet ist.

Eine Vorrichtung dieser Art ist aus der US-A-4,823,807 bekannt. Auf dem Bildschirm werden die jeweiligen Frequenzen und Amplituden der aufgenommenen Tonsignale als Funktion des Grades der Abwinkelung des untersuchten Gelenks dargestellt und der Arzt kann aus diesen Darstellungen Rückschlüsse über den jeweiligen Krankheitsbild ziehen. Ferner besteht die Möglichkeit der aufgenommenen Daten zu speichern und einen Vergleich der aufgenommenen Daten mit früheren Daten vorzunehmen.

Bei der bekannten Vorrichtung muss der Patient soviel Muskelkraft aufwenden, dass ein Drehmoment um die Gelenkachse konstant bleibt. Dies stellt eine Beschränkung des Verfahrens dar, da der Patient körperlich im Stande sein muss, die erforderliche Muskelkraft aufzubringen. Dies ist beispielsweise nach einem chirurgischen Eingriff nicht immer leicht möglich.

Auch andere Ansätze existieren Erkrankungen anhand von Tonsignalen zu untersuchen. Obwohl die vorliegende Erfindung sich hauptsächlich mit Gelenk- und Weichteilerkrankungen bzw. deren therapeutische Behandlung und Rehabilitation sowie entsprechende Vorrichtungen handelt, soll vorerst der Begriff "Osteoporose" und die Bedeutung der Krankheit kurz erläutert werden, um die Erfindung besser erläutern zu können.

Laut verschiedenen Angaben gibt es in Deutschland ca. 6-8 Millionen Osteoporosekranke. Durch ca. 1/4 Million Frakturen, insbesondere Schenkelhalsfrakturen, beziffern sich die Kosten der Behandlung der Osteoporose und der Folgen der Osteoporose auf eine zweistellige Milliardensumme.

Die Knochensubstanz unterliegt einem physiologischen Umbau. Die Zellen, die die Knochenmassen aufbauen und z.B. nach einer Fraktur reparieren, sind Osteoblasten. Sie nehmen Calcium und andere Mineralien aus dem Blut und binden sie in den Knochen. Auf diese Weise wird die Knochensubstanz fester. Die andere Art der Zellen, die Knochensubstanz abbauen und überschüssiges Knochenwachstum verhindern, nennt man Osteoklasten.

Im physiologischen Zustand besteht Gleichgewicht zwischen beiden Vorgängen. Mit zunehmenden Alter gewinnt der Knochenabbau die Oberhand. Einige Quellen geben an, daß es ab dem 25. Lebensjahr zu verstärktem Knochenabbau sowohl bei Männern als auch bei Frauen kommt.

Einfach gesagt versteht man unter dem Begriff Osteoporose einen porösen, morschen Knochen. Medizinisch muß jedoch von einer relativ seltenen Krankheit, der Osteomalazie, unterschieden werden. Sowohl Osteoporose als auch Osteomalazie fallen medizinisch unter den Gattungsbegriff der Osteopenie.

Unabhängig von Ursachen der Osteoporose bei Frauen und Männern kommen die gleichen, bildgebenden Verfahren zur Anwendung. Am häufigsten wird die Knochendichte mittels Computertomographie untersucht. Diese Methode beinhaltet jedoch viele Nachteile, wie z. B. Strahlenbelastung und Beeinflussung der Meßwerte in der unteren LWS durch die Veränderungen wie Spondylophyten, Osteochondrose usw.

Die zunehmend häufigste Methode ist die Messung mit Ultraschall. Die Patienten werden jedoch hier in orthopädische und gynäkologische Praxen gedrängt, um sich einer Ultraschalluntersuchung zu unterziehen. Die Genauigkeit dieser Messung ist jedoch bis jetzt sehr unzuverlässig, wie aus der Literatur und der täglichen Praxis bekannt ist. Die Knochendensitometrie mittels Ultraschall erfolgt in einem Frequenzbereich von 200 kHz bis 1 Mhz und wird seitens der gesetzlichen Krankenkassen zusätzlich eingeschränkt. Das geschieht aus zwei Gründen: zum einen aufgrund der relativ hohen Kosten von durchschnittlich 80 - 130 DM pro Untersuchung und zum anderen aufgrund der oben angesprochenen Unzuverlässigkeit dieser Methode.

Die Röntgendiagnostik zeigt mit Sicherheit osteoporotische Veränderungen erst ab ca. 30 Prozent Knochendichteabnahme. Die Densitometrie ist in solchen Fällen nicht mehr nutzbar. Mit entsprechender Behandlung fängt man auch ohne diese Untersuchung an.

Aufgabe der vorliegenden Erfindung ist es, eine Vorrichtung der eingangs genannten Art anzugeben, mit dem bzw. mit der man eine Reihe von Überprüfungen und Diagnosen im Zusammenhang mit dem anatomischen Zustand von Gelenken des Körpers eines Menschen oder eines Tieres kostengünstig und zuverlässig durchführen kann, und zwar ohne medizinische Eingriffe durchführen oder Proben entnehmen zu müssen.

Zur Lösung dieser Aufgabe zeichnet sich die erfindungsgemäße Vorrichtung dadurch aus, dass eine Einrichtung oder eine Programmierung vorgesehen ist, die ausgelegt ist, um Signale zu erzeugen, die den jeweiligen Grad der Abwinklung des untersuchten Gelenkes bei der Durchführung der Bewegung angeben, und dass der Rechner ausgelegt ist, um das Ergebnis der Signalanalyse in Form einer Animation auf dem Bildschirm darzustellen.

Die Erfindung beruht auf der Erkenntnis, daß Tonsignale, die durch Bewegung eines Gelenks in Bestandteile des Körpers eines Menschen oder eines Tieres verursacht werden, bei entsprechender Auswertung Rückschlüsse auf den Zustand der jeweiligen Bestandteile ermöglichen. Beispielsweise erlaubt die Auswertung Aussagen über die Materialeigenschaften, die Mineralisierung, die Ultrastruktur, die Kollagenstruktur, die Mikrostruktur und die Geometrie des Knochens. Vor allem aber erlaubt die Auswertung Aussagen über den anatomischen Zustand von Gelenken und diesen zugeordneten Weichteilen.

Die Auswertung kann erfindungsgemäß durch Filterung und Spektralanalyse erfolgen und umfaßt auch in vielen Fällen einen Vergleich mit Referenzwerten bzw. mit Referenzmustern. Dabei ist die angewandte Filterung üblicherweise als Tiefpaßfilterung, beispielsweise im Bereich von bis zu ca. 10 kHz zu verstehen. Hierdurch wird die Bandbreite begrenzt. Die Analyse und Auswertung wird vorteilhaft mittels eines Computerprogramms durchgeführt.

Es kann sich bei den Bewegungen des Gelenks um passive oder aktive Bewegungen handeln. Unter aktiven Bewegungen versteht man, daß der Patient selbst ein ausgewähltes Gelenk bewegt. Bei passiven Bewegungen wird dagegen eine Einrichtung benützt, die die Bewegung eines ausgewählten Gelenks erzwingt. Vorteilhaft bei der Anwendung von aktiven Bewegungen ist, daß der Patient durch technische Geräte nicht verunsichert oder verängstigt wird.

Andererseits kann bei passiven Bewegungen die Geschwindigkeit und Amplitude der Bewegung genau und wiederholbar vorgegeben werden, so daß hier die Ergebnisse von verschiedenen Patienten besser miteinander vergleichbar sind.

Ein Sonderfall der passiven Bewegungserzeugung liegt darin, daß der Arzt oder eine andere Person das Gelenk manuell bewegt.

Die Erfindung ist jedoch nicht hierauf beschränkt. Beispiele für weitere mögliche Anwendungen sind:

1. Kontrolle der Knochenkonsolidation (Kallusbildung nach Frakturen). Bei konservativer Therapie, d.h. ohne operative Versorgung, ist diese Methode mit Sicherheit anwendbar.
Bei osteosynthetisch versorgten Knochenbrüchen werden sich bestimmte Probleme mit zuverlässigen Referenzen aufgrund der Vielfalt der Methoden und der verschiedene Sorten von Metallimplantaten ergeben.
Vorteil: Röntgenkontrollen bei konservativ versorgten Frakturen können auf ein Minimum reduziert werden.

2. Signalanalyse beider Beine bei diffusen Beschwerden, z. B. Unterschenkel bei jungen Patienten. Bekannt ist, daß bei dieser Patientengruppe statistisch häufig primäre Knochenläsionen (Tumore) entstehen.
Ein Vorteil liegt darin, daß das Meßergebnis als Entscheidungshilfe verwendet werden kann, ob weitere Untersuchungen angebracht sind. Beispielsweise kann die Abwesenheit eines Unterschieds zwischen den beiden unteren Extremitäten als Grund zum Verzicht auf weitere bildgebende Verfahren gelten.
Bei einer Differenz der Signalanalyse müssen unbedingt weitere Untersuchungen wie Röntgen oder Szintigraphie durchgeführt werden.

3. Bei vielen anderen Knochenkrankheiten, z.B. Plasmozytom (bösartiger Tumor), kommt es häufig zum Befall der Schädelknochen ("Mottenfraß"). Das Abklopfen und die Signalanalyse des Schädels können bei entsprechenden Referenzen plastische und lytische Knochenveränderungen ausschließen oder bestätigen. Die Folge sind weitere Untersuchungen wie Röntgen oder Computertomographie.

4. Die o.g. Methode kann vor allem bei Gelenkkrankheiten und Weichteilläsionen anzuwenden ist. Zu diesen gehören Arthrosen (Knorpelverlust). Jeder Mensch wird im Laufe des Lebens davon betroffen. Von Vorteil ist die Möglichkeit der Unterbringung von mehreren Sensoren an verschiedenen Orten. Dadurch kann z.B. Gonarthrose (Kniegelenkverschleiß) genau differenziert werden. Es ist anzunehmen, daß anhand der Signalanalyse festgestellt werden kann, ob es sich um Verschleiß des Innengelenkspaltes oder Außengelenkspaltes handelt. Auch der Verschleiß der Rückseite der Kniescheibe könnte diagnostiziert werden.
Bezüglich der Patella (Kniescheibe) gibt es immer diagnostische Probleme, und zwar sowohl klinisch als auch mit bildgebenden Verfahren wie NMR. Mittels der durch die erfindungsgemäß durchgeführte Signalanalyse kann festgestellt werden, ob Probleme der Rückseite der Kniescheibe überhaupt vorhanden sind. Die entsprechenden Geräusche (Spektren) lassen sich bestimmten Krankheiten zuordnen. Von Chondropathia patellae, Chondromalazie patellae bis zu Retropatellararthrose (Knorpelverlust auf der Rückseite der Kniescheibe bzw. des Patellagleitlagers).

5. Die Methode ist auch bei verschiedenen Weichteilerkrankungen anwendbar, z.B.:

- Meniskopathien
- Ausmaß der Achillodynie (das Knirschen des "Paratenons") zusätzlich zur sonographischen Abschätzung der pathologischen Situation.
- Coxa saltans (schnappende Hüfte).
- Labrumläsion der Schultergelenke.
- Rotatorenmanschettenläsionen der Schulter, vor allem Engpässe (M. supraspinatus)

- Discus triangularis Läsionen - bis jetzt große diagnostische Probleme auch mittels NMR der Handgelenke.

Möglicherweise kann man durch die erfindungsgemäß durchgeführte Signalanalyse zusätzliche diagnostische Vorteile gewinnen.

6. Eine weitere denkbare Anwendung wäre die Darstellung des Carpaltunnelsyndromes (sehr häufige Erkrankung - Einengung des N. medianus im Carpaltunnel des Handgelenkes). Diese Krankheit verursacht tausende von Operationen in Deutschland pro Jahr.
Die Diagnose wird üblicherweise mittels NLG (Nervengleitgeschwindigkeitsmessungen) des N. medianus gestellt. Theoretisch scheint mit Analyse des z.B. Klopfgeräusches des Carpaltunnels die Diagnosestellung möglich zu sein (das Abklopfen des Carpaltunnels wird in der Orthopäde als Hoffmann-Tinel-Test bezeichnet. Bei diesem Test wird jedoch das Ergebnis des Klopfens nicht akustisch ausgewertet, sondern durch das subjektive Schmerzempfinden des Patienten). Möglicherweise können hier entsprechende Referenzen gefunden werden.
Besonders bevorzugte Ausführungsformen der Erfindung sind den Ansprüchen zu entnehmen.

An dieser Stelle soll der Vollständigkeit halber auf die US-A-5,213,555 hingewiesen werden, die ein System beschreibt, das es ermöglicht Rennen auf Kraftmaschinen in Fitnessstudios zu veranstalten. Auf einem Videomonitor kann der Verlauf des Rennens dargestellt werden, beispielsweise mit einer Darstellung der jeweiligen von einzelnen teilnehmenden Radfahrern zurückgelegten Strecken. Diese Darstellung hat aber nichts mit der Auswertung von Tonsignalen von krankhaften Gelenkteilen zu tun.

Auch die US-A-5,931,763 soll kurz erwähnt werden. Diese Schrift befasst sich ebenfalls mit Übungsgeräten in Fitnessstudios und umfasst Monitore die Auswertungen zu den durchgeführten Übungen und Angaben zu weiteren Trainingsstufen anzeigen. Auch diese Darstellungen haben nichts mit der Auswertung von Tonsignalen von krankhaften Gelenkteilen zu tun.

Die Erfindung wird nachfolgend näher erläutert anhand von Ausführungsbeispielen unter Bezugnahme auf die beigefügten Zeichnungen, in denen Fig. 1 bis 3 eine Methode zur Diagnose von Osteoporose darstellen, während die weiteren Figuren 4 bis 10 Beispiele der Erfindung angeben.

Es zeigen:

Fig. 1 ein schematisches Diagramm zur Erläuterung des erfindungsgemäßen Verfahrens,

Fig. 2 ein schematisches Diagramm zur Erläuterung einer möglichen Variante der Signalauswertung bei dem Verfahren gemäß Fig. 1,

Fig. 3A das Ergebnis einer erfindungsgemäßen Messung an einem gesunden, männlichen Patienten im Alter von 42 Jahren,

Fig. 3B das Ergebnis einer erfindungsgemäßen Messung an einem an Osteoporose erkrankten männlichen Patienten im Alter von 44 Jahren,

Fig. 3C das Ergebnis einer erfindungsgemäßen Messung an einer weiblichen, an Osteoporose erkrankten Patientin im Alter von 64 Jahren,

Fig. 3D das Ergebnis einer erfindungsgemäßen Messung an einer älteren, weiblichen, an ausgeprägter Osteoporose erkrankten Patientin im Alter von 72 Jahren,

Fig. 3E das aufgenommene Tonsignal bei dem gleichen Patienten wie in Fig. 3A betrachtet und

Fig. 3F das aufgenommene Tonsignal bei der gleichen Patientin wie in Fig. 3C betrachtet.

Fig. 4A bis 4C schematische Darstellungen eines gesunden menschlichen Kniegelenks,

Fig. 4D eine schematische Darstellung eines unter medialer Gonarthrose leidenden menschlichen Kniegelenks,

Fig. 5 eine schematische Darstellung einer erfindungsgemäßen Tonaufnahmevorrichtung,

Fig. 6A eine Kniegelenkorthese,

Fig. 6B eine erfindungsgemäße Modifikation der Orthese der Fig. 6A,

Fig. 7A eine Spektralanalyse von Tonsignalen eines gesunden menschlichen Kniegelenks nach den Fig. 4A bis 4C,

Fig. 7B ein Histogramm entsprechend der Fig. 7A,

Fig. 8A eine Spektralanalyse von Tonsignalen eines unter medialer Gonarthrose leidenden menschlichen Kniegelenks nach Fig. 4D,

Fig. 8B      ein Histogramm entsprechend der Fig. 8A,

Fig. 9A      eine schematische Darstellung einer für die Erfindung verwendbaren Kraftmaschine,

Fig. 9B      eine schematisches Diagramm zur Erläuterung einer erfindungsgemäßen Einstellung einer Kraftmaschine nach Fig. 9A und

Fig. 10      eine schematische Darstellung einer erfindungsgemäßen Vorrichtung mit Animation.

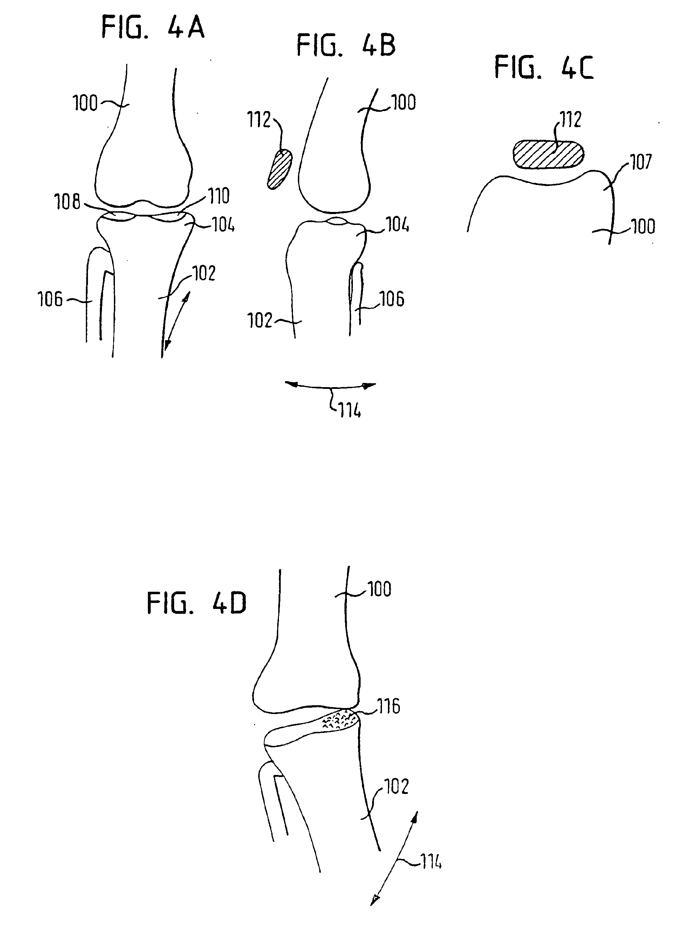

Durch die Bewegungen und inneren Reibungen entstehen im menschlichen Körper Geräusche. Diese Geräusche können einiges über den inneren Zustand des Körpers aussagen. In dieser Beschreibung wird insbesondere die Aufmerksamkeit auf die Knochen- und Gelenkgeräusche gerichtet. Es wird zunächst eine prinzipielle Methodik zur Bestimmung und Auswertung dieser Geräusche beschrieben, die bei der Diagnose von Osteoporose in der Praxis des Anmelders untersucht worden ist und es wird - aufbauend auf dieser Beschreibung - anschließend im bezug auf die weiteren Figuren 4 bis 10 der vorliegenden Erfindung näher erläutert. Bei einer Diagnose von Osteoporose geht es in erster Linie um die Auswertung des Klopfgeräusches (longitudinale Vibrationen) die mit einem gleiche Impulse liefernden Gerät ausgelöst werden. Auf die ausgewählte Meßstelle (Tibiakopf anatomisch kaum Weichteildeckung), klopfte man ca. 5 cm kaudal.

Geräusche sind grundsätzlich longitudinale Luftvibrationen, die von unseren Ohren wahrgenommen werden können. Nach dem Empfang können sie vom Gehirn in einer sehr breiten Palette analysiert werden. Das menschliche Gehirn ist fähig, aus einem Geräusch bestimmte Muster wiederzuerkennen - Mustererkennung - oder bestimmte Töne daraus auszusortieren (Filtern).

Alle diese Fähigkeiten stellen in Wirklichkeit sehr komplizierte Mechanismen dar, die uns erst beim Versuch der künstlichen Realisierung dieser Fähigkeiten bewußt werden.

Genau diese Probleme haben jahrzehntelang Wissenschaftler aus allen Bereichen beschäftigt. Im Laufe der Jahre sind stets mächtigere Tools (Systeme) zur Analyse bzw. Mustererkennung von Tonsignalen entstanden. Man sehe dies an der Leistung der heutigen Spracherkennungsprogramme, die sich drastisch verbessert hat.

Bevor mit der Analyse des Signals begonnen wird, sollen die Signalgewinnung und die richtige Aufbereitung der Signale beschrieben werden.

Die uns interessierenden Geräusche können auf zwei Arten entstehen.

Die eine Möglichkeit ist durch Bewegung. Bewegt man ein Gelenk in eine bestimmte Richtung, kommt es zu einer inneren Reibung im Gelenk, was man hören kann.

Die zweite Möglichkeit setzt einen äußeren Stoß auf den Knochen voraus, der ihn zum Schwingen bringt, was wiederum mitgehört werden kann.

Es muß nebenbei auf Geräusche der Weichteile hingewiesen werden, z. B. der Muskulatur im Schulterbereich, der speziellen anatomischen Struktur im Handgelenk usw. Solche Geräusche können ebenfalls erfindungsgemäß ausgewertet werden und zu verbesserter Diagnose der entsprechenden Bestandteile führen.

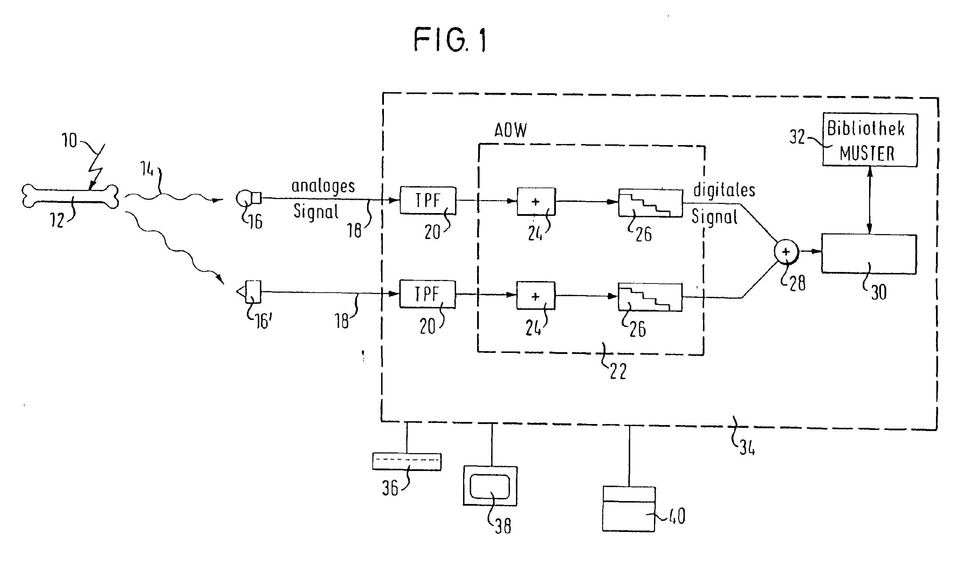

Die erfindungsgemäße Verarbeitung der Geräusche ist schematisch in Fig. 1 dargestellt und kann bei allen anatomischen Geräuschen bzw. Tonsignalen unabhängig von deren Entstehungsursache verwendet werden.

Der erste Schritt bei der Signalaufbereitung ist das Erfassen der Tonsignale. Dabei werden diese in elektrisch meßbare Werte umgewandelt.

Für solche Aufgaben werden meistens Sensoren eingesetzt. Ein Sensor ist ein Bauelement, das eine mechanische Größe aufnimmt und nach entsprechender Umwandlung zu einem elektrischen Signal führt. Bei dieser elektrischen Größe kann es sich sowohl um die Spannung als auch um den Strom oder sogar um die Kapazität handeln. Der Sensor ist aber ein analoges Bauelement, was bedeutet, daß er zeitkontinuierliche Signale ausgibt, die alle beliebigen Werte haben können. Für die Signalanalyse müssen sie aber zunächst digitalisiert werden. Das wird mit einem Analog-Digital-Wandler (ADW) bewerkstelligt. Allerdings muß bei der Umwandlung das Signalspektrum mit Hilfe eines Tiefpaßfilters begrenzt werden, damit die Nyquis'sche Abtastbedingung erfüllt bleibt.

Man kann sich unter einem ADW einen Abtaster vorstellen, der das analoge Signal in festen Zeitintervallen abtastet bzw. abliest und anschließend durch einem Quantizierer den analogen Wert in einen digitalen Wert umwandelt. Die Voraussetzung für diesen Vorgang ist durch die Nyquissche Abtastbedingung gegeben, die eine Mindestabtastrate (Ts) von doppelten Bandbreite (W) des Signals verlangt.

$$T_s \le \frac{1}{2W}.$$

Es soll noch weiterhin darauf hingewiesen werden, daß man bei der Untersuchung mehrere Sensoren an verschieden Orten unterbringen kann.

Nach der Vorbereitung des Digitalsignals kann mit der eigentlichen Signalanalyse begonnen werden.

Dafür steht eine Vielfalt von Möglichkeiten zur Verfugung, die je nachdem zum erwünschten Ergebnis führen können. Es ist zu betonen, daß diese Tools, was die Komplexität und den Rechenzeitverbrauch angeht, sehr unterschiedlich sind. Beim Wählen der richtigen Methode muß dementsprechend ein Kompromiß gefunden werden.

An dieser Stelle sollen zwei Methoden der Signalanalyse vorgestellt werden, die in Praxis sehr oft eingesetzt werden.

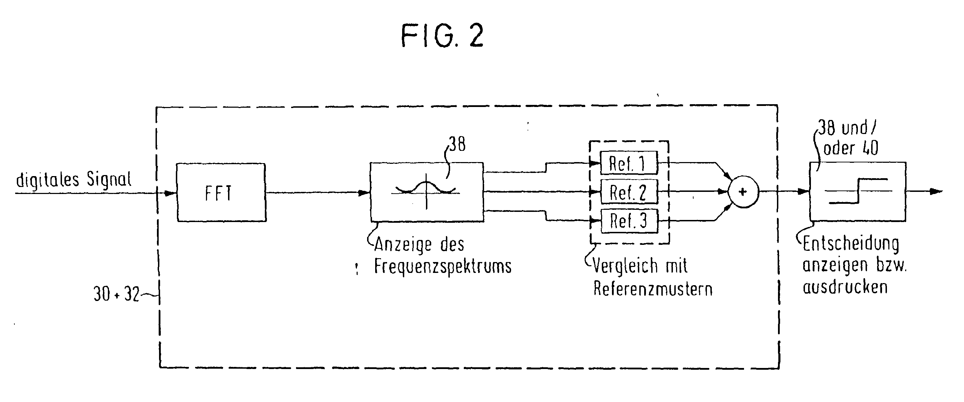

Die erste Methode, die gleichzeitig die einfachere Methode ist, besteht darin eine Fast Fourier-Transfomation (FFT) durchzuführen und anschließend das so erhaltene Frequenzspektrum mit anderen Referenzspektren zu vergleichen (siehe Fig. 2).

Danach wird das Signal, das ursprünglich im Zeitbereich angegeben ist, nach der Gleichung in den Frequenzbereich transformiert. Aus diesem Grund wird die Methode auch Spektralanalyse genannt.

Der Vorteil dieser Methode besteht darin, daß man sehr rasch und einfach die Intensität aller Spektren bzw. Frequenzen eines Zeitintervalls beobachten kann.

Die Fourier-Analyse (FFT) wird auf dem Gebiet der elektrischen Nachrichtentechnik häufig angewandt und befaßt sich mit einer mathematischen Methode zur Analyse von komplexen Wellenformen oder Signalen in eine Reihenfolge von einfachen Oberwellenfunktionen, deren Frequenzen jeweils ein ganzes Vielfache der Grundfrequenz sind. Es gilt:

$$Fn(f) = \int_{\alpha}^{+\alpha} f(t) \; e^{-iwt} \; . dt,$$

In der oben angegebenen Gleichung bedeutet f(t) das Tonsignal, das als Funktion der Zeit (t) aufgenommen wird. w bedeutet die Grundfrequenz und Fn(f) die Fourier-Transformation, d.h. das Ergebnis der Fourier-Analyse in Form der Reihenfolge der Grundwelle des Tonsignals und deren Oberwellen mit den jeweiligen Amplituden und Phasenverschiebungen. $\alpha$ entspricht unendlich und die andere Symbole haben die übliche Bedeutung.

Falls die gesuchte Information in der Spektrumverteilung des Signals steckt, kann man mit FFT am besten diese Informationen herauslesen. Das Beispiel eines osteoporotischen Knochens ist so ein Fall. Man kann durch Angabe des Spektrums des Signals schnell feststellen, ob es sich dabei um ein dumpfes Geräusch handelt oder nicht.

Die zweite Methode ist die der neuronalen Netze, die sehr mächtig und leistungsintensiv ist und sehr verschiedene Variationen besitzt. Für die Untersuchung der komplexeren Muster im Signal und unüberschaubare Mustererkennungen im Zeitbereich sollte man auf diese Methode zugreifen.

Neuronale Netze sind logische Schaltungen, die der Funktionalität unseres Gehirns gleichen. Wie es beim Gehirn der Fall ist, sind sie lernfähig und erst durch eine Lernphase können sie eingesetzt werden. Das spielt für die Auswahl der passenden Lernmuster eine große Rolle.

Für die Auswertung der Analysen benötigt man eine Referenz, womit man das angemessene Signal vergleicht und daraufhin seine Entscheidung trifft. Die Art dieser Referenz kann von Fall zu Fall sehr unterschiedlich sein. Sie muß aber mit der Methodenanalyse übereinstimmen.

Nachdem zwei Möglichkeiten der Signalanalyse vorgestellt wurden, soll ein praktisches Beispiel anhand der Figuren 1 bis 3 mit Bezug auf die Ermittlung von Osteoporose angegeben werden, die eine verhältnismäßig einfache und praktische Anwendung darstellt, wie in Figur 1 gezeigt wird. Das zu untersuchende Tonsignal wird durch eine äußere Einwirkung 10 auf einen Knochen 12, beispielsweise auf die Tibia oder auf den Tibiakopf, durch den Schlag eines Arzthammers verursacht. Dadurch kommt der Knochen 12 in Vibration und gibt ein Tonsignal 14 ab. Dieses Tonsignal 14 wird mit einem Mikrophon 16 aufgenommen und als elektrisches Signal an einen Tiefpaßfilter 20 weitergeleitet. Nach dem Tiefpaßfilter läuft das Signal in einen Analog-Digitalwandler 22 bestehend aus einem Abtaster 24 und einem Quantizierer 26. Das Ausgangssignal des Analog-Digitalwandlers 22 ist ein Digitalsignal, das einer Einrichtung 30 zugeführt wird, die eine FFT-Analyse durchführt, die Angaben zu dem Frequenzspektrum des Tonsignals 14 liefert, d.h. die vorhandene Grundfrequenz, deren Oberwellen und die jeweiligen Amplituden angibt.

Das Mikrophon ist selbst eine Art Sensor. Auch andere Sensoren, beispielsweise Piezosensoren, kommen in Frage und es können mehrere Sensoren beispielsweise 16 und 16' verwendet werden. Wenn andere Sensoren wie 16, 16' verwendet werden, können deren jeweiligen digitalisierten Signalen in den Eingangskanal 28 der die Fourier-Transformation durchführenden Einrichtung 30 mit entsprechender Kennung eingespeist werden. Bei manchen Untersuchungen kann es auch vorteilhaft sein, die Signale von mehreren Sensoren auszuwerten, um besondere Diagnosen zu stellen, beispielsweise am Kniegelenk. Die Kennung erlaubt es der nachfolgenden Einrichtung 30 die Signale der verschiedenen Sensoren diesen jeweils zuzuordnen.

Der Tiefpaßfilter begrenzt die Bandbreite auf einen gewünschten Bereich, beispielsweise von 0 bis etwa 10 kHz.

Die Einrichtung 30 ist außerdem dazu ausgelegt, das Frequenzspektrum des Digitalsignals mit einem Referenzmuster oder mit mehreren Referenzmustern zu vergleichen, die in einem als "Bibliothek" bezeichneten Speicher 32 gespeichert sind. Diese Referenzmuster in der Bibliothek 32 basieren auf früheren Messungen, beispielsweise vom gleichen Patienten, oder von einem Patienten gleichen Geschlechts, gleichen Alters, gleichen Gewichts oder gleichen Körperbaus. Sie können alternativ hierzu aus Referenzmustern bestehen, die aus Meßwerten gebildet sind, die von einer Gruppe von Patienten stammen, die mit dem jeweiligen Patienten vergleichbar sind, beispielsweise nach den Kriterien gleiches Geschlecht, gleiches Alter, gleiches Gewicht oder gleicher Körperbau, wobei solche Referenzmuster auch in verschiedenen Klassen je nach Grad der Osteoporose unterteilt werden können.

Um den Vergleich durchzuführen ist es vorteilhaft die Daten zu komprimieren wozu ein der bekannten Datenkompressionsverfahren benutzt werden kann. Die Durchführung des Vergleichs ist in Fig. 2 schematisch dargestellt. Aufgrund des durchgeführten Vergleichs gelingt es festzustellen, ob der jeweilige Patient Osteoporose hat und wenn ja auch den Grad der Erkrankung.

Die Einrichtungen 20, 22, 24, 26, 28, 30 und 32 können durch einen Computer 34 gebildet werden, wie durch den Kasten in Fig. 1 mit dem gleichen Bezugszeichen angedeutet. Der Computer kann beispielsweise über eine Tastatur 36 für die Eingabe von Patientendaten, Zeitangaben und Befehle, einem Bildschirm 38 zur Darstellung der Ergebnisse der Messungen und des herangezogenen Referenzmusters bzw. der herangezogenen Referenzmuster sowie einen Drucker 40 zum Ausdrucken der Ergebnisse verfügen.

In einer praktischen Version der Erfindung kann das Tonsignal an die Soundkarte eines Computers weitergeleitet werden, die die Filterungs- und Digitalisierungsaufgaben und anschließend die Analyse direkt erledigt.

Die Grundidee dieser akustischen Untersuchung der Osteoporose basiert auf dem Gedanken, daß der menschliche Knochen wie jedes andere Objekt je nach eingeschlossenem Inhalt "anders" klingen soll. Das heißt, je poröser der Knochen ist, desto dumpfer soll er beim Klopfen klingen. Diese Eigenschaft kann sehr gut anhand einer Spektralanalyse beobachtet werden. Das entspricht einer Häufung der Niederfrequenzanteile des Spektrums. Nach der FFT des Signals kann die Verschiebung des Spektrums im niedrigen Frequenzbereich sichtbar gemacht werden.

Anhand von Untersuchungen an genau vordiagnostizierten Patienten und gesunden Probanden werden jetzt einige Beispiele angegeben, die beweisen daß das oben genannte Verfahren bei Osteoporose einwandfrei funktioniert.

Anschließend wird kurz anhand eines gesunden Probanden und vordiagnostizierten Osteoporosepatienten mittels z.B. Osteodensitometrie die Plausibilität der vorgestellten Methode gezeigt.

Im weiteren werden die theoretischen Möglichkeiten der diagnostischen Signalanalyse bzgl. der großen Gelenke dargestellt.

Ferner wird auf die theoretischen Möglichkeiten der Diagnostik der Weichteilerkrankungen hingewiesen.

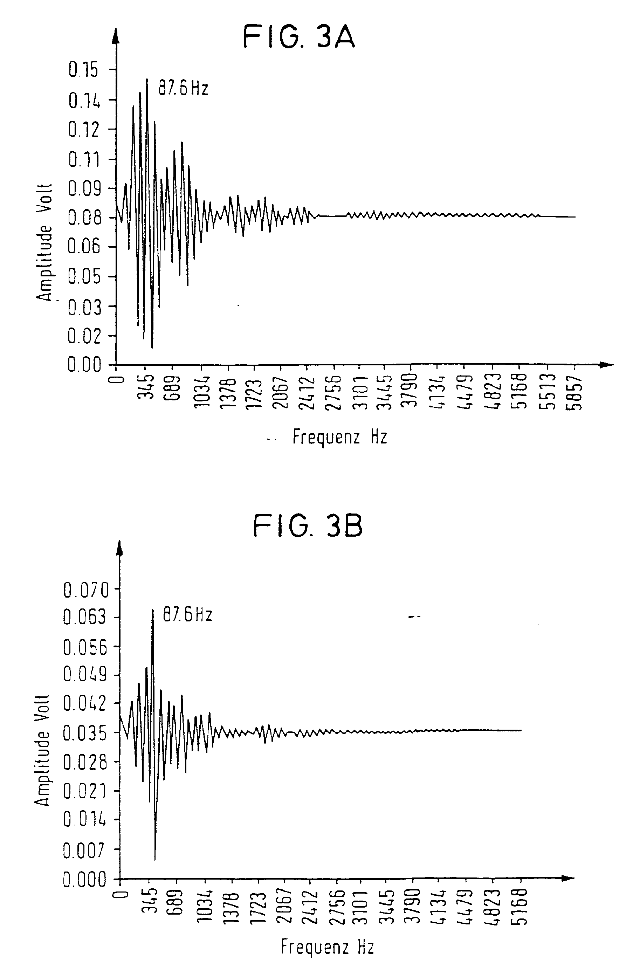

Fig. 3A zeigt das Ergebnis der Messung an einem gesunden Knochen eines 42jährigen Mannes.

Die x-Achse gibt die Frequenz in Hz an, während die y-Achse einen Spannungswert in V liefert. Die Höhe der Grundlinie, d.h. der Anfang an der y-Achse in Volt hängt von der Tonstärke ab und scheint nicht patientenabhängig zu sein und ist daher wahrscheinlich unwichtig für die Auswertung. Die Amplituden bei den einzelnen Frequenzen, d.h. die relativen Amplituden der Frequenzspitzen untereinander, liefern jedoch wichtige Informationen.

Um ein sauberes Signal zu bekommen, wurde mehrmals auf die Tibia geklopft. Beim zehnten Mal wurde das Signal ausgewertet. Wie oben beschrieben, Impuls mit "Arzthammer" auf ventrale Tibia proximal ca. 5 cm unterhalb des Kniegelenkspaltes, Messung am Tibiakopf.

Fig. 3B zeigt den Knochen eines osteoporotischen männlichen Patienten (44 Jahre). Die Messung wurde aufgenommen, wie oben im Zusammenhang mit Fig. 3A beschrieben ist, jedoch hier beim ersten Klopf. Obwohl der Patient im ähnlichen Alter ist wie der gesunde Patient, an dem die Messung gemäß Fig. 3A durchgeführt wurde, gibt es sichtbare Verschiebungen des Spektrums in den niedrigeren Frequenzbereichen. Der Unterschied ist deutlich zu sehen: der 44jährige Patient wurde mittels Osteodensitometrie vorher diagnostiziert. Die Standardabweichung betrug 1,75.

Die Osteoporose kann man als manifest einstufen.

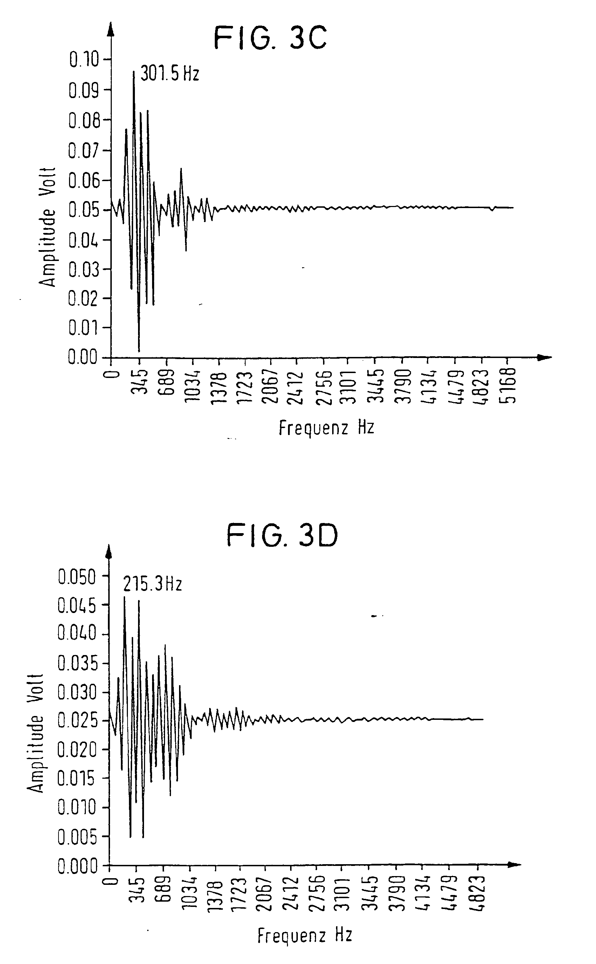

Fig. 3C zeigt den Knochen einer 64jährigen Patientin mit mittelgradiger Osteoporose.

Laut Befund der Densitometrie beträgt die Knochendichteabnahme 10 - 15 Prozent. In diesem Fall wurde das Klopfgeräusch beim vierten Mal ausgewertet.

Fig. 3D zeigt die Messung einer 72jährigen Patientin mit schwerer Osteoporose und mehreren durchgemachten Frakturen einschließlich Kompressionsfrakturen der WS. In diesem Fall wurde der Ton beim zweiten Mal gemessen. Das sichtbare Ergebnis ist sehr eindrucksvoll.

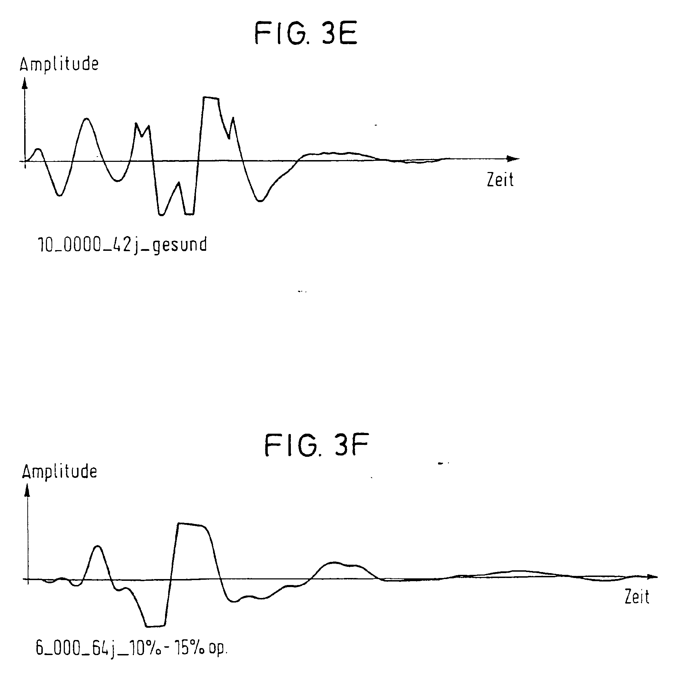

Figuren 3E und F zeigen den Unterschied der Amplitude zwischen einem gesundem Knochen (Patient in Fig. 3A) und einem osteoporotischem Knochen (Patientin in Fig. 3C) noch vor der Rechnerauswertung.

Auffällig ist die breite der Amplitude bei einem Osteoporotiker. Bei einem Gesunden scheint die Amplitude eher zeitverkürzt und peakartig zu sein.

Die o. g. Beispiele wurden mittels eines normalen Mikrophons untersucht bzw. aufgenommen. Es ist anzunehmen, dann bei der Benutzung der speziellen Sensoren die Ergebnisse noch ausdrucksvoller und genauer erscheinen werden.

Das beweist, daß die vorgestellte Methode prinzipiell funktioniert.

Weitere Untersuchungen bei Gelenken und Knochen haben gezeigt, daß tatsächlich viele Geräusche einigen bestimmten Krankheiten zugeordnet werden können.

Ein wichtiger Teil der Arbeit ist die Herstellung zuverlässiger Referenzen, denn die Zuverlässigkeit der getroffenen Entscheidungen hängt sehr stark von der richtigen Wahl der Referenzen ab.

Gesichert ist jedoch auch ohne Referenzen die Unterscheidung zwischen gesunden und osteoporotischen Knochen. Allein aus diesem Grund dürfte diese Methode eine breite Akzeptanz finden. Durch sicheren Ausschluß der Osteoporose können unnötige Röntgenuntersuchungen und diverse Densitometrien vermieden werden.

Die Ersparnisse der Ausgaben der Patienten und der Krankenkassen können enorm sein.

Das o.g. Konzept der Knochendichtemessung (Osteodensitometrie mittels Messung der longitudinalen Wellen-Ton) wird sich in Zukunft problemlos auf Software übertragen lassen. Das betrifft auch die Entwicklung der speziellen Programme für die Untersuchung der Gelenke und Weichteile, wie oben genannt. Die zukünftige Softwareherstellung wird vermutlich in kurzer Zeit die Osteoporoseuntersuchung in jeder Praxis, die mit Computer ausgestattet ist, ermöglichen. Allein die einfache Bestätigung oder Ausschließung einer Osteoporose ohne großen Kosten- und Arbeitsaufwand ist als enormer Schritt für die tägliche Tätigkeit des Arztes in der Praxis zu sehen. Eine Internetübertragung kann auch bei der Untersuchung hinzugezogen werden. Beispielsweise kann eine Arztpraxis über das Internet Referenzmuster von anderen Quellen (Arztpraxen und dergleichen) abrufen. Als eine Alternative kann der Computer 34 das Ergebnis einer aktuellen Messung (mit oder ohne vorherige Auswertung) über das Internet an eine Auswertungs- bzw. Computerzentrale schicken, wo die Auswertung durchgeführt wird. Das Ergebnis der Auswertung kann dann online dem Computer 34 zur Anzeige und/oder zum Ausdrukken zugeleitet werden. Auf diese Weise werden die Messungen einer großen Anzahl Praxen zur Bildung qualitativ hochwertiger Referenzmuster zur Verfügung stehen, vor allem wenn sie mit dem Ergebnis weiterer Messungen, wie beispielsweise Osteodensitometriemessungen an den gleichen Patienten verknüpft werden.

Es ist auch durchaus denkbar, daß die Referenzmuster, auf ein Speichermedium wie beispielsweise CD gespeichert, einzelnen Arztpraxen und anderen Institutionen verfügbar gemacht werden.

Die angeheftete Referenzliste enthält Angaben zu Literaturquellen, die Informationen über die Signalanalyse im allgemeinen liefern und können gegebenenfalls zur Hilfestellung herangezogen werden. Obwohl sich die bisherige Beschreibung mit Messungen an Menschen befaßt, kann das erfindungsgemäße Verfahren bzw. die erfindungsgemäße Vorrichtung im Zusammenhang mit Tieren angewendet werden, vor allem bei Rennpferden, bei Diagnosen beispielsweise bezüglich Renntauglichkeit und Bruchgefahr.

Fig. 4 zeigt eine schematische Darstellung des rechten Kniegelenks eines Menschen, wobei die Fig. 4A die Darstellung von vorne zeigt, mit dem Femurknochen 100, dem Tibia 102 mit Tibiakopf 104 und dem Fibula 106. Die Bezugszeichen 108 und 110 zeigen auf den Außenmeniskus bzw. den Innenmeniskus.

Fig. 4B zeigt die gleiche Darstellung von der Knieaußenseite gesehen und zeigt zusätzlich die Kniescheibe oder Patella 112.

Die Fig. 4C zeigt ebenfalls die Kniescheibe 112, hier in einer Darstellung entsprechend der Längsachse des Femurknochens 100, wobei das Knie in der gebeugten Stellung axial gezeigt ist und die Femurgleitrolle 107 ersichtlich ist. Es handelt sich bei dieser Darstellung um die Darstellung eines physiologisch "idealen" Kniegelenkes, d.h. eines Gelenk in gutem Zustand. Der Doppelpfeil 114 zeigt die Bewegungen an, die beim Beugen und Strecken des Kniegelenks auftreten.

Dagegen zeigt die Fig. 4D ein ähnliches Gelenk, bei dem der Zustand einer medialen Gonarthrose vorliegt, d.h. ein Verschleiß des Innengelenkspaltes, der bei 116 dargestellt ist. Diese Arthrose 116 macht sich im Geräuschspektrum bei einer Biegung des Kniegelenks entsprechend dem Pfeil 114 bemerkbar, der auch in der schematischen Darstellung gemäß Fig. 4D ersichtlich ist.

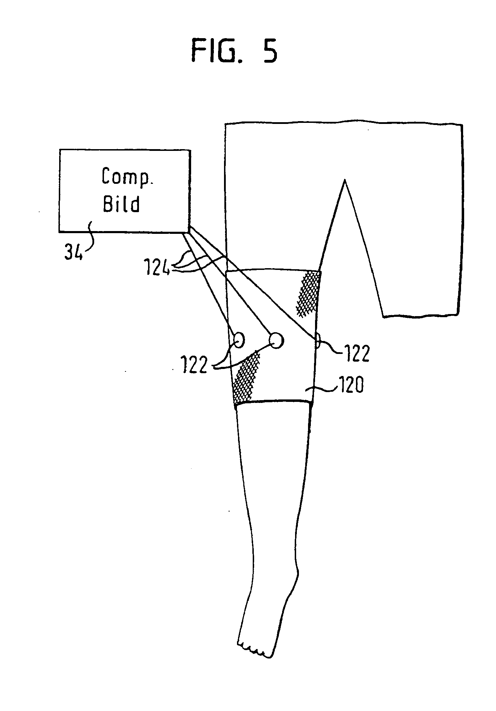

Eine Vorrichtung zur Aufnahme der Geräusche, die bei Biegung des Kniegelenks entstehen, ist in der Fig. 5 dargestellt. Es handelt sich hier um einen elastischen Strumpf 120, der über das Kniegelenk gezogen wird und welcher in diesem Beispiel mit drei Mikrophonen 122 ausgestattet ist, die über Leitungen 124 mit einem Computer, wie 34, gekoppelt sind. Anstelle drei Mikrophone zu verwenden, kann man auch mit einem einzigen Mikrophon arbeiten und es können auch andere akustische Signale aufnehmende Sensoren verwendet werden. Auch ist es nicht erforderlich, die Mikrophone über Signalleitungen 124 mit dem Computer zu verbinden, sondern es könnte eine drahtlose Übertragung gewählt werden. Hierfür wird beispielsweise eine Sendereinheit mit entsprechenden Batterien in den Strumpf eingebaut, so daß die Signalübertragung an den Computer mittels Infrarotsignalen oder unter Anwendung einer anderen drahtlosen Übertragung erfolgt, wobei natürlich eine entsprechende Empfängereinheit dem Computer 34 zugeordnet werden muß. Bei diesem Beispiel wird das Kniegelenk entweder vom Patienten selbst (aktiv) bewegt oder es wird vom Arzt oder einer Hilfsperson manuell, d.h. passiv, bewegt.

Am besten ist es jedoch, wenn die Mikrophone in einer Hilfsvorrichtung oder Orthese eingebaut sind, die unabhängig von den anatomischen Gegebenheiten des Gelenkes am Kniegelenk befestigbar sind. Der Strumpf 120 kann einen Teil dieser Hilfsvorrichtung bilden oder die Mikrophone können an die Hilfsvorrichtung in einer anderen Art und Weise angebracht werden. Sinn der Hilfsvorrichtung ist es sicherzustellen, daß die Tonaufnahme immer bei einer gleichartigen Bewegung des Kniegelenks erfolgt, so daß die aufgenommenen Tonsignale reproduzierbar sind, wodurch einerseits ein Vergleich der aufgenommenen Signale des jeweiligen Patienten bzw. Probanden mit Referenzwerten, die unter gleichen Bedingungen aufgenommen wurden, ermöglicht wird und andererseits auch ein Vergleich mit anderen Aufnahmen für den gleichen Patienten bzw. Probanden möglich ist, d.h. ein Vergleich zwischen Aufnahmen, die zu verschiedenen Zeitpunkten erfolgt sind.

Eine Form der Hilfsvorrichtung wäre eine Schiene, die der natürlichen Bewegung des Gelenks folgt oder erzwingt. Beispielsweise kann für das Kniegelenk eine Schiene verwendet werden, wie sie in Krankenhäusern für die postoperative Bewegung des Kniegelenks eines Patienten benutzt wird, der sich beispielsweise einen Kreuzbandriß zugezogen hat, der operativ behoben wurde. Bei einer solchen Vorrichtung liegt der Oberschenkel des Patienten auf einer ersten Schiene, während sein Unterschenkel auf einer zweiten Schiene liegt, die über ein polyzentrisches Gelenk mit der ersten Schiene gekoppelt ist und gegenüber der ersten Schiene von einem Motor angetrieben wird, um Bewegungen um das polyzentrische Gelenk zu erzwingen und entsprechende Bewegungen des Kniegelenks des Patienten durchzuführen. Diese Schiene kann zum Zwecke der Erfindung auch ohne Motor betrieben werden, wenn die zweite Schiene vom Patienten selbst (aktiv) oder von einer Hilfsperson (passiv) um das polyzentrische Gelenk bewegt wird. Eine solche Vorrichtung könnte eine CAPM Orthese (Continuous Active/Passive Motion Orthese) genannt werden.

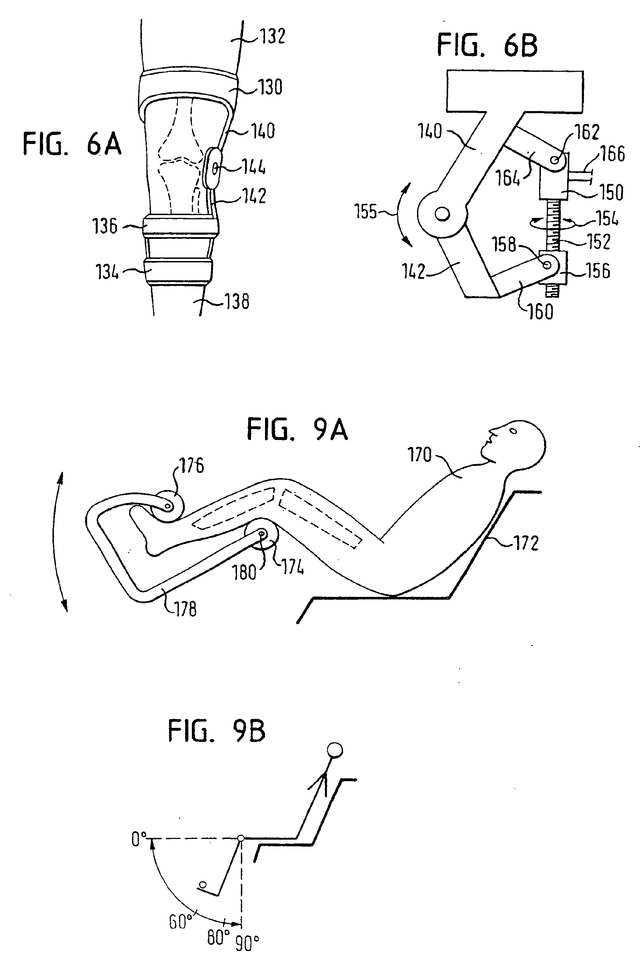

Eine andere mögliche Auslegung der Hilfsvorrichtung ähnelt einer an sich bekannten Kniegelenkorthese, beispielsweise wie in Fig. 6A schematisch dargestellt. Es handelt sich hier um eine Knieorthese wie sie von der Firma Medi Bayreuth in Deutschland erhältlich ist und unter der Bezeichnung "Monarch GA" verkauft wird. Diese Orthese besteht aus einem ersten Fixierband 130, das um den Oberschenkel 132 des Probanden befestigt wird, beispielsweise mit Hilfe eines Klettverschlusses sowie aus zwei unteren Bandagen 134 und 136, die am Unterschenkel 138 befestigt werden und ebenfalls als Beispiel mit einem Klettverschluß versehen werden können. Vom oberen Befestigungsband 130 bzw. den oberen Befestigungsbändern 130, wenn zwei vorgesehen sind, was auch möglich ist, und den unteren zwei Bändern 134 und 136 erstrecken sich im Prinzip starre Arme 140 und 142, die aneinander an einem Gelenk 144 befestigt sind. Das Gelenk führt eine polyzentrische Bewegung aus, entsprechend der Bewegung des menschlichen Kniegelenks und ist mit dem Kniegelenk des Patienten bzw. des Probanden ausgerichtet. Diese Anordnung der beiden Arme 140 und 142 kann auch auf der linken Seite des Beins in Fig. 6A wiederholt werden, was hier nicht gezeigt ist. Um passive Bewegungen des Kniegelenks zu erzwingen, kann an diese Orthese ein Motor 150 befestigt werden, wie in Fig. 6B gezeigt. Dieser Motor 150 treibt eine Gewindespindel 152 in zwei alternative Drehrichtungen an, die durch den Doppelpfeil 145 gezeigt sind. Dabei erstreckt sich die Gewindespindel, wie in Fig. 6B gezeigt, durch eine Mutter 156, die schwenkbar um die Achse 158 an einem Arm 160 befestigt ist, der fest mit dem Arm 142 verbunden ist.

Der Motor 150 selbst ist um die Achse 162 an einen weiteren Arm 164 schwenkbar angelenkt, der mit dem Arm 140 fest verbunden ist. Der Motor kann vom Computer 34 aus über die Leitungen 166 angesteuert werden, um die Spindel 152 wechselweise in die eine oder andere Pfeilrichtung 154 zu drehen. Hierdurch wird eine polyzentrische Bewegung des Kniegelenks entsprechend der Auslegung des Gelenks 144 erzwungen und diese Bewegung ist vom zeitlichen Ablauf gesehen, also auch in seiner Amplitude, Geschwindigkeit und Häufigkeit, genau über den Computer 34 vorgebbar, so daß die gleiche Bewegung bei allen Probanden bzw. Patienten sichergestellt ist. Für Patienten mit Kniebeschwerden kann gegebenenfalls über den Computer 34 bzw. eine gesonderte Motorsteuerung eine unterschiedliche Amplitude der Bewegung des Kniegelenkes vorgegeben werden, so daß sie nicht unnötig Schmerzen oder gar Schäden erleiden müssen. Auch kann gegebenenfalls die Geschwindigkeit der Bewegungen und die Wiederholfrequenz entsprechend unterschiedlich gewählt werden. Der Motor 150, die Spindel 152 sowie die diese tragenden Arme 160, 164 und die zugeordneten Teile 156, 158, 162 sind vorzugsweise so ausgelegt, daß sie leicht entfernbar sind. Beispielsweise können sie auf der Orthese durch Aufklipsen befestigt werden, so daß sie "mit einem Klick" entfernt werden können. Auf diese Weise kann die Hilfsvorrichtung leicht in eine normale Orthese umgewandelt werden.

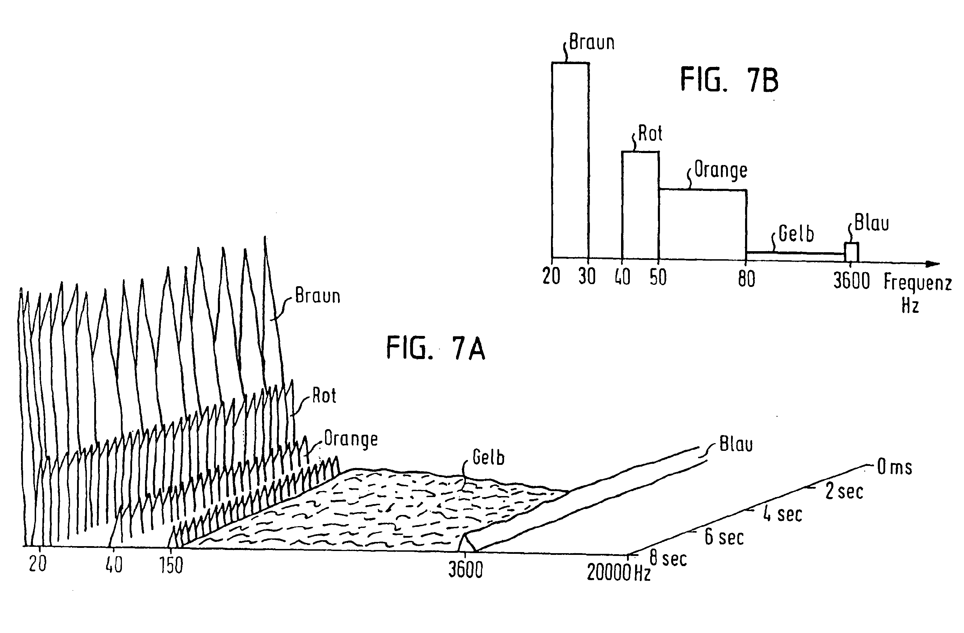

Die Fig. 7A zeigt nun in einer dreidimensionalen Darstellung das Ergebnis der Aufnahme von Kniegelenkgeräuschen bei einem Probanden mit einem Kniegelenk in gutem Zustand entsprechend den Fig. 4A bis C.

Diese Aufnahme wurde gemacht unter Anwendung eines Programms zur akustischen Signalanalyse, die als Werkzeug für die verschiedensten akustischen Signalanalysen kommerziell erhältlich ist. Es handelt sich um das Wave Lab Programm, das von Herrn Steinberg bzw. von Miro Computer Products AG, Cham, Deutschland, erhältlich ist.

Die Untersuchung wird mit einem speziellen, am Computer angeschlossenen Mikrophon durchgeführt. Die Geräusche werden an bestimmten Gelenkstellen aufgenommen. Für die genaue Untersuchung ist allerdings, wie schon erwähnt, eine spezielle Schieneneinrichtung bzw. Orthese notwendig.

Die Auswertung erfolgt bis jetzt mit einem Standardprogramm der Frequenzanalyse, z.B. Wave Lab-Steinberg (siehe oben). Es werden sowohl mathematische Daten gespeichert, wie Tonhöhe, Loudness, Peak, als auch eine schematische Darstellung benutzt. Ein ganz unauffälliges Gelenk ohne pathologische Geräusche der Weichteile, der Knorpel und anderen wird schematisch in Form einer Frequenzanalyse dreidimensional dargestellt. Es werden drei Parameter gezeigt: Frequenz in Hertz, Amplitude und die Zeit von 0 bis Ende der Untersuchung (hier nach 8 Sekunden). Das Gelenk wird mehrfach bewegt, durchschnittlich fünfmal. Nach Fast-Fourieranalyse bearbeitet der Computer die akustische Gelenksituation. Bei einem unauffälligen Gelenk, wie in diesem Beispiel, sind die farbig markierten Pegel, wie schematisch dargestellt, sehr regelmäßig. Die Amplitude der Frequenz zwischen 20 Hz bis ca. 30 Hz ist am höchsten (die erste dreidimensionale Struktur von links. d.h. bei der Signalfarbe Braun). Die nächste Struktur ab ca. 40 Hz bis 50 Hz ist durchschnittlich um die Hälfte kleiner, d.h. bei der Signalfarbe Rot. Dann folgt eine wieder um ein Drittel kleinere Struktur bei ca. 50 Hz bis 80 Hz, d.h. bei der Signalfarbe Orange. Die nächsten Pegel ab 150 Hz sind äußerst niedrig und nur angedeutet, d.h. bei der Signalfarbe Gelb. Bei der Frequenz von ca. 3.600 Hz gibt es wiederum eine kleine Anhebung, die mit der Signalfarbe Blau angedeutet wird.

Der zeitliche Verlauf der Pegel ist symmetrisch, keine Unterschiede von 0 bis 1 Sek. und von 0 bis z.B. 8 Sek. (je nach Länge der Untersuchung).

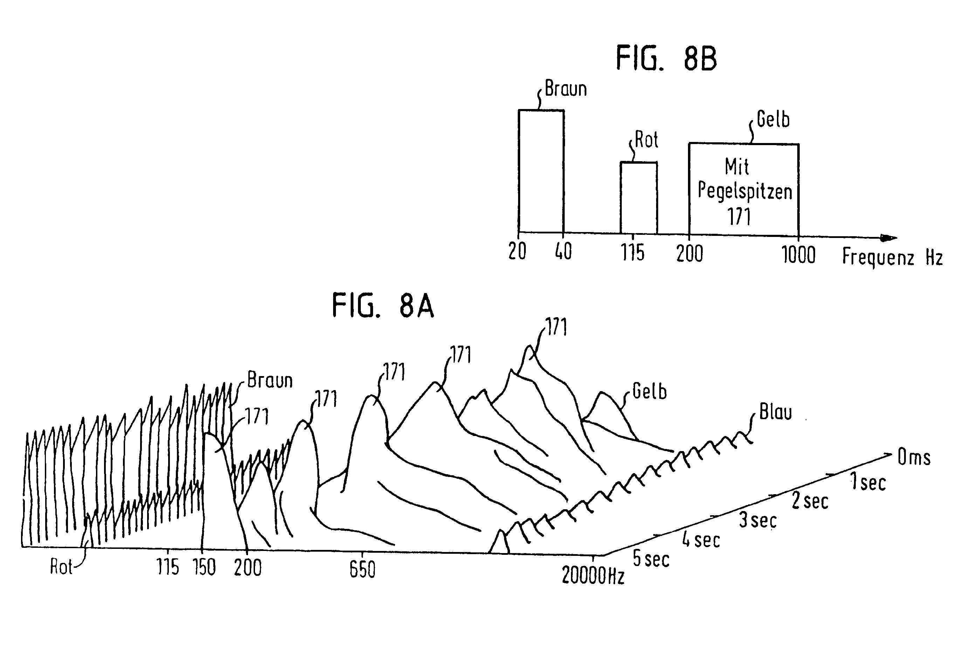

Das pathologische Gelenk zeigt sich, abhängig von der konkreten Diagnose, anders. Ein Beispiel ist in Fig. 8A für ein Kniegelenk angegeben, das eine mediale Gonarthrose aufweist. Es besteht eine sehr genaue Korrelation zwischen funktioneller Diagnose und schematischer Darstellung bzw. mathematischen Daten. Bei z.B. medialer Gonarthrose zeigen sich wie aus Fig. 8A ersichtlich, deutlich niedrigere Pegel bei 20 Hz und 40 Hz, dagegen sind vereinzelte, teilweise hohe und unregelmäßige Pegel 171 bei 115 Hz vorhanden. Ausschlaggebend ist die Häufigkeit und Höhe der Pegel zwischen 200 Hz bis ca. 1000 Hz (hier gelb markiert). Die Schwere der Arthrose beziehungsweise die Reibungsvorgänge der beschädigten Knorpel-Knochenstrukturen wird in diesem Bereich deutlich gezeigt. Weniger von Bedeutung sind in diesem Fall die Strukturen bei 3.500 Hz, 8.000 Hz und ca. 18.000 Hz. Bei Entlastung (entweder manuell und mit der schon beschriebenen Schiene) der betroffenen Gelenkteile oder mit der bekannten Orthese (Monarch GA) ändern sich diese Parameter. Bei diesem Programm werden die Pegel (Amplituden) der Frequenzen zwischen 200 Hz bis 650 Hz deutlich kleiner. Die Amplituden der niedrigen Frequenzen werden dagegen höher. Daraus können diagnostische Schlüsse (wie schon beschrieben) gezogen werden.

Ein ganz anderes Bild und ganz andere, jedoch konkrete mathematische Parameter zeigt der Computer bei Weichteilerkrankungen. Z.B. bei Tendovaginitis stenosans des Daumens (schnellender Daumen) sind die Pegel bei 20 Hz und 40 Hz äußerst niedrig. Die gelben Pegel sind auch nicht vorhanden. Das Geräusch ist sehr laut und hat ganz andere Eigenschaften als das Gelenkgeräusch.

Die Fig. 7B zeigt wie diese dreidimensionale Frequenzanalyse, beispielsweise in Form eines Balkendiagramms wiedergeben werden kann. Bei einer Computerdarstellung kann man beispielsweise wahlweise das Histogramm oder die dreidimensionale Darstellung wie in Fig. 7A abrufen (oder beides gleichzeitig) und diese Darstellungen können auch farblich dargestellt werden, so daß unterschiedliche Farben den jeweiligen Frequenzbereichen zugeordnet sind, wie dies in Fig. 7A und 8A angedeutet ist.

Somit ermöglicht die vorliegende Erfindung durch Auswertung der Geräusche, die von einem Gelenk ausgehen eine eindeutige Diagnose des Zustandes des Gelenkes und es ist zu erwarten, daß unterschiedliche Signalauswertungen so differenziert ausfallen, daß man alle wesentlichen medizinischen Probleme im Bereich des Kniegelenks, auch im Zusammenhang mit den Weichteilen, wie die Menisken durch unterschiedliche Frequenzspektren der aufgenommenen Signale diagnostizieren kann. Nicht nur die Frequenzunterteilung liefert nützliche Informationen über die jeweils vorliegende Krankheit bzw. Zustandsänderung des Knies. Es ist beispielsweise aus der Fig. 8A ersichtlich, daß die Frequenzen im Bereich 150 bis 1000 Hz wieder diskrete Spitzen aufweisen, die auch charakteristisch für ein Knie mit Arthrose entsprechend der Fig. 4D sind.

Untersuchungen in der Praxis des Anmelders haben gezeigt, daß durchaus eine berechtigte Hoffnung besteht, für alle einschlägigen Kniegelenkerkrankungen eine Diagnose stellen zu können, und zwar in einer Art und Weise, die für den Patienten im Prinzip nicht schmerzhaft ist und auf jeden Fall unter Umständen manche unnötigen medizinischen Eingriffe verhindern kann. Das gleiche gilt auch für alle anderen Gelenkpartien des Menschen, wo Bewegungen von Knochen oder Weichteilen stattfinden.

Die Erfindung kann aber nicht nur zur Diagnose verwendet werden, wie im Zusammenhang mit den Fig. 4 bis 8 beschrieben, sondern sie kann auch zur therapeutischen Behandlung des jeweiligen Gelenks eingesetzt werden. Um ein Beispiel hierfür zu nennen, wird nochmals auf die Darstellung gemäß Fig. 6A hingewiesen.

Es wurde bereits oben zum Ausdruck gebracht, daß es sich hier um eine Orthese der Firma Medi von Bayreuth handeln kann. Mit einer solchen Orthese sind in den Bändern 130, 136, 124 und im Bereich des Gelenkes 144 Luftkammern eingebaut, die mittels einer Luftpumpe aufgepumpt werden können. Es ist auf diese Weise oder mit einer im Bereich des Gelenks 144 angeordneten Schraube möglich, einen seitlichen Druck auf das Kniegelenk auszuüben, so daß z. B. bei Abnutzungserscheinungen der Gelenkpartie im Bereich des Innenmeniskus 108 der Kniegelenkspalt an dieser Stelle leicht geöffnet werden kann, dafür aber der Kniegelenkspalt im Bereich des Außenmeniskus eher mehr belastet wird. Durch diese Entlastung dieser beschädigten Stelle des Kniegelenkes durch Öffnung des Kniegelenkspaltes tritt hier eine Entlastung auf, die sich im Geräuschsignal widerspiegelt. Somit kann der Arzt bzw. Orthopädiemechaniker gegebenenfalls unter Zuhilfenahme der Vorrichtung gemäß Fig. 6B, aber auch ohne Anwendung des Motors das Kniegelenk bewegen und die Frequenzanalyse betrachten, bis diese zeigt, daß Geräusche nicht mehr im Bereich des Innenmeniskus 108 entstehen. Sobald dies erreicht ist, weiß er, daß eine ausreichende Entlastung des Kniegelenkes erreicht ist und der Patient kann mit dem Gerät in der beabsichtigten Art und Weise sich bewegen, während sich die betroffene Gelenkpartie erholt oder durch besondere Medikamente oder Behandlungen in einen besseren Zustand gebracht wird. Nach der Einstellung des Gelenkspaltes kann der Motor 150 und die zugeordneten Teile von der Orthese entfernt werden, wie vorher angedeutet. Während der Therapie läuft der Patient herum, d.h. erledigt seine tägliche Arbeit mit angezogener Orthese, jedoch ohne Motor 150.

Wenn gesagt wird, daß das Gelenk, ohne den Motor 150 zu benutzen, bewegt wird, so ist dies in dem Sinne zu verstehen, daß der Patient selbst sein Knie biegt, beispielsweise indem er leichte Kniebeugungen durchführt oder geht, das wären dann aktive Bewegungen des Kniegelenkes, oder der Patient kann im Sitzen oder Liegen sein Kniegelenk manuell von einer anderen Person bewegen lassen, das wären dann passive Bewegungen.

Wenn die Therapie beendet ist, d.h. eine anfängliche Verbesserung des Zustands des Kniegelenks eingetreten ist, fängt dann die Phase der Rehabilitation an. Hier wird der Patient entweder von einem Physiotherapeuten betreut oder kann selbst in ein Fitnessstudio gehen und die dort verwendbaren Kraftmaschinen anwenden, die für die Stärkung des Kniegelenkes und der diesem zugeordneten Muskulatur gedacht sind. Ein solches Gerät ist schematisch in Fig. 9 gezeigt. Hier sitzt der Patient 170 auf einem Sitzteil 172 eines Gerätes mit einer fest angeordneten, jedoch drehbaren Rolle 174, welche in der Kniebeuge des Patienten angeordnet ist und seine Knie abstützt, dabei greifen die Füße des Patienten unterhalb eines zylindrischen stabartigen Teils 176, das über ein Gestell oder einen Rahmen 178 drehbar um die Achse 180 der Rolle 174 angeordnet ist. Der Patient muß versuchen das Teil 176 mit seinem Fuß anzuheben, so daß sich das Gestell 178 um die Drehachse 180 schwenkt, dabei wäre eine Schwenkbewegung im Bereich zwischen 0 und 90° üblich, wie aus Fig. 9B ersichtlich ist. Solche Geräte sind so ausgelegt, daß der Widerstand gegen diese Drehbewegung einstellbar ist. Nun kann man sich vorstellen, daß der Patient wiederum den Strumpf gemäß Fig. 5 anzieht und daß während dieser Bewegung an der Kraftmaschine die akustischen Signale wiederum aufgenommen werden. Bspw. wird sich dann zeigen, z.B. aufgrund einer Arthrose im Bereich des Knies, daß in dem Winkelbereich von z.B. 60 bis 80° eine unangenehme Reibung im Gelenk auftritt, so daß gerade in diesem Bereich der Bewegung der Widerstand, der von der Kraftmaschine ausgeht, möglichst klein sein soll, während in dem Bereich der Bewegung zwischen 90 und 80° bzw. zwischen 60 und 0° keine schlechten Stellen des Kniegelenkes belastet werden, so daß hier mit einem erhöhten Widerstand gearbeitet werden kann, so daß sich der erwünschte Zustand einstellt. Mit anderen Worten wird der Widerstandsverlauf der Kraftmaschine jeweils Patienten spezifisch eingestellt. Die Rehabilitation des Kniegelenkes durch Aufbau der Muskulatur schreitet dann wie erwünscht fort, ohne daß die beschädigten Partien des Kniegelenkes, die es zu rehabilitieren gilt, belastet werden. Die so ausgelegten Kraftmaschinen sind nicht nur für Rehabilitation zu verwenden, sondern auch in der Freizeit zum Fitness-Training.

Auch hier ist das Beispiel anhand eines Kniegelenks gegeben worden. Selbstverständlich kann jede andere Art von Kraftmaschine, die besondere Muskelpartien trainieren soll, entsprechend ausgebildet werden, um das jeweils zugeordnete Gelenk des Patienten zu rehabilitieren oder zu trainieren.

Anstelle die Sensoren am Kniestumpf zu montieren, könnten diese auch eventuell in die Kraftmaschine selbst eingebaut werden und es könnte sogar die Kraftmaschine so ausgelegt werden, daß sie unter Auswertung der aufgenommenen Tonsignale automatisch sich selbst so einstellt, daß beschädigte Gelenkstellen verschont bleiben und schneller rehabilitiert werden. Wenn eine einmalige Einstellung der Kraftmaschine für einen bestimmten Patienten ermittelt ist, so kann diese Einstellung patientenspezifisch gespeichert und für zukünftige Übungen an der Maschine beibehalten werden, so daß es nicht notwendig ist, bei jeder erneuten Benutzung der Kraftmaschine die Einstellungen neu zu ermitteln oder daß der Patient erneut einen Kniestrumpf mit Mikrophon anzieht.

Die Erfindung geht aber auch noch weiter. Man kann sich vorstellen, daß die Erfindung zur Einstellung bei Skistiefeln oder anderen in den verschiedensten Sportarten verwendeten Geräten benutzt wird. Dies wird anhand von neuen Skistiefeln erläutert.

Es ist bekannt, daß gute Skifahrer, wie z.B. Hermann Maier, sich weit in die Vorlage begeben können, d.h. das Knie extrem abbiegen können, ohne daß sie hierdurch Schmerzen oder Gelenkverschlechterungen im ausgeprägten Maße erleiden. Dagegen gibt es andere Skifahrer, die älter sind oder eine schwächere Muskulatur oder bereits beschädigte Knie haben, die eine so extreme Vorlage wie Hermann Maier nicht durchführen können. Gut wäre es, wenn in diesem Falle die Skistiefel so ausgelegt werden könnten, daß sie bei einem bestimmten Vorlagebereich, entweder Sperren oder einen Widerstand gegen eine weitergehende Vorlage, d.h. eine weitere Kniebiegung aufweisen. Beispielsweise könnte dies durch gesonderte Federanordnungen im Stiefel oder durch Austauschen von Schienbeinschalen erfolgen, die sich unterschiedlich am Vorderteil des Stiefels abstützen und daß somit die Vorlage des Fahrers begrenzen. Nun kann man sich vorstellen, daß der Skifahrer, der sich ein neues Paar Stiefel besorgen möchte, sich in ein Geschäft begibt, dort einen Strumpf entsprechend der Fig. 5 anzieht und Kniebeugen selbst durchführt, d. h. unter Belastung (beispielsweise mit Gewichten auf den Schultern) aktive Bewegungen durchführt. Auch passive Bewegungen kämen in Frage, d.h. die Vorrichtung gemäß Fig. 6A und B könnten auch hier zur Anwendung gelangen. Wenn nun die akustische Signalanalyse zeigt, daß der jeweilige Skifahrer leicht eine Vorlage bis 30° erreichen kann, daß aber eine weitergehende Vorlage über diesen Winkel hinaus zu einer unerwünschten Belastung des Kniegelenkes führt - ermittelt durch die sich ändernden Kniegeräusche - so weiß das jeweilige Sportgeschäft, daß die für ihn geeigneten Stiefel so eingestellt werden müssen oder so ausgewählt werden müssen, daß sie bei einer Vorlage von 30° sperren oder eine Bewegung über 30° hinaus wesentlich erschweren. Es wird hierdurch verhindert, daß der Skifahrer in einer Vorlagestellung fährt, die für ihn schädlich ist.

Um dies zu realisieren, ist es natürlich notwendig, was auch bei den bisher beschriebenen Ausführungsformen sinnvoll ist, die Gelenkgeräusche entsprechend dem Grad der Abbiegung des Gelenkes winkelspezifisch auszuwerten, was auch erfordert, daß eine Einrichtung vorgesehen ist, die den Grad der Abbiegung mißt.

Werden die Signale unter Anwendung einer Orthese entsprechend der Fig. 6B aufgenommen, kann beispielsweise eine Winkelmeßeinrichtung im Gelenk 144 aufgenommen werden oder die Ansteuersignale für den Motor 150 können zu diesem Zweck herangezogen werden. Beispielsweise könnte der Motor solange in eine Richtung gedreht werden, bis die Spindel 152 möglichst aus der Mutter 156 herausgeschraubt ist, so daß sich das Gelenk in der gestreckten Position befindet. Wenn hier der Anschlag erreicht ist, wird dies als 0° Biegung angenommen. Wenn der Motor 150 dann beispielsweise als Schrittmotor ausgelegt ist, so kann anhand der insgesamt ausgeführten Schritte der Motorbewegung und aus Angaben zu der Steigung des Gewindes der Gewindespindel 152 und der Geometrie der Orthese eine genaue Ermittlung des jeweiligen Beugungswinkels des Gerätes für jede Bewegung des Schrittmotors und der Spindel ermittelt werden.

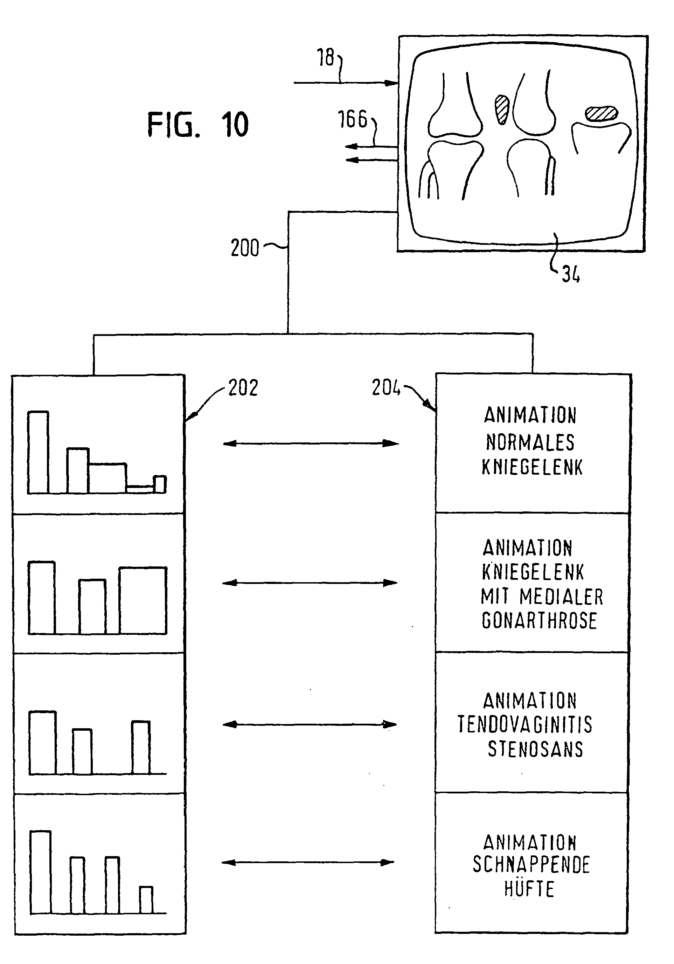

Eine Besonderheit der vorliegenden Erfindung liegt darin, daß eine Animation möglich ist, die die Diagnose des jeweils vorliegenden Problems erleichtert und auch für den Patienten bzw. Probanden leicht nachvollziebar ist. Anhand des Kniegelenkes kann man sich jetzt vorstellen, daß die Bilder, die in Fig. 4A bis C gezeigt sind, schematisch im Bildschirm des Computers 34 erscheinen wie in Fig. 10 gezeigt und daß bei der Bewegung des Kniegelenkes die entsprechende Bewegung der Gelenkteile im Bildschirm durch eine entsprechende Animation gezeigt werden. Auch hier können die Winkelsignale, die oben besprochen werden, zum Zwecke der Animation nützlich sein. Auf jeden Fall kann die Signalauswertung herangezogen werden, um auf der Bildschirmanimation die Bereiche anzuzeigen, wo aufgrund der Signalauswertung Problemstellen zu finden sind. Beispielsweise könnten die entsprechenden Stellen am Bildschirm durch eine blinkende Darstellung oder durch besondere Farbgebung hervorgehoben werden, so daß der Arzt dem Patienten beispielsweise sagen kann "schau her, Dein Innenmeniskus ist lädiert" oder "Du leidest unter Arthrose". Diese Hervorhebung der lädierten Stellen kann entweder bei einer statischen Darstellung der Gelenkbilder gemäß Fig. 4A bis C oder bei gleichzeitiger Bewegung der Gelenkglieder oder, wenn das Problem nur in einem bestimmten Winkelbereich auftritt, bei einer Darstellung, die diesen Winkelbereich entspricht, beispielsweise bei einem Beugewinkel des Knies von 30°, was vor allem entsprechend der Darstellung der Fig 4B angezeigt werden könnte. Eine Möglichkeit, eine solche Darstellung zu realisieren, ist schematisch in der Fig. 10 gezeigt. Hier sehen wir den oben angesprochenen Computer 34 und es ist schematisch dargestellt, daß der Computer über eine Datenleitung 200 mit zwei Speicherbereichen 202 und 204 kommuniziert.

Diese Speicherbereiche 202 und 204 können im Computer selbst vorhanden sein, oder sie können in externen Speichern beispielsweise Fernspeichern realisiert werden. Die Datenleitung 200 könnte beispielsweise auch als eine Internetverbindung verstanden werden, so daß der Computer 34 beim Arzt in der Praxis steht, während die Speicherbereiche, auf die der Computer 34 zugreifen kann, an einem entfernten Ort stehen.

Im Speicher 202 sind dann beispielsweise eine Reihe von Histogrammen entsprechend den Fig. 7B und 8B gespeichert, während im Bereich 204 entsprechende Darstellungen des Knies mit Darstellung des jeweiligen Problems enthalten sind. D.h., daß der Computer 34 aufgrund der jeweiligen Aufnahmen beim Arzt, die dem Computer über die Leitung 18 zugeführt werden, eine Spektralanalyse durchführt und auf die entsprechenden abgespeicherten Referenzhistogramme bzw. Muster aus dem Bereich 202 zurückgreift und die dazu passenden Bilder aus dem Speicher 204 abruft und im Bildschirm darstellt. Wenn das auf dem Bildschirm gezeigte Gelenk im Rahmen der Animation entsprechend der Bewegung des untersuchten Gelenks bewegt wird, so kann diese Bewegung im Bildschirm wesentlich langsamer erfolgen, damit die Problemstellen für den Betrachter des Bildschirms leichter zu erkennen sind. Obwohl nur vier Speicherbereiche für Frequenzanalysen und Animationsmöglichkeiten (202A, 202B, 202C und 202D bzw. 204A, 204B, 204C und 204D) in Fig. 10 gezeigt sind, werden in der Praxis eine große Zahl von unterschiedlichen Frequenzanalysen und Animationsmöglichkeiten bzw. Diagnosen gespeichert. Fig. 10 ist rein beispielhaft zu verstehen und gilt nicht nur für Kniegelenke, sonder für alle anderen in Frage kommenden Gelenke.

Zusammenfassend wird betont, daß ein Computerprogramm mit schematischer Darstellung des untersuchten Gelenkes relativ einfach zu erstellen wäre. Man kann jedes Gelenk, analog zu dem oben beschriebenen Schema, schematisch darstellen. Es könnten schematisch Problemzonen, wie z.B. Reibungsvorgänge, Weichteileinklemmungen, Meniskuseinklemmungen usw., gezeigt werden. Dadurch könnte eine konkrete Person ihr Gelenk bei der Bewegung beobachten. Ähnlich könnte man Weichteile, wie die Einklemmungsstelle im Sehnenverlauf, darstellen. Dies wäre ein sehr wichtiger Aspekt bei Rehabilitation, Leistungssport usw. Die Biofeeback-Rolle ist auch nicht zu vergessen. Bei z.B. pathologischen Druck der Kniescheibe auf die Oberschenkelgleitrolle zwischen z.B. 60° und 80° könnte ein Kraftgerät speziell eingestellt werden (analog zu Cybex 2-Gerät). Dadurch könnte man die pathologischen Reibungsvorgänge in diesem Bereich vermeiden. Ein weiteres Beispiel zur Benutzung dieser Methode (Datenbank, entsprechende Vorrichtung, entsprechende Gelenkschiene mit Sensoren bzw. Mikrophonen) wäre die Anpassung der Skischuhe in Bezug auf das Kniegelenk. Die Mehrheit der Skifahrer leidet an retropatellaren Schmerzen, wie z.B. Chondropathia patellae. Die akustische Bestimmung der Schuhvorlage könnte abhängig von pathologischen Signalen aus dem Kniegelenk stattfinden. Dies wäre von großer Bedeutung sowohl beim Leistungsskifahrer als auch beim Freizeitskifahrer. Bei einem physiologischen, nicht beschädigten Gelenk ist natürlich eine sehr weite/weiche Vorlage ohne Einschränkung möglich. Bei Pathologie soll eine schwere Vorlage, abhängig vom akustischen Signal, angewandt werden. Dies ist die einzige Methode, wo aus akustischem Signal ein bildgebendes Verfahren in der Orthopädie zustande kommt. Der Vorteil dieser Methode ist eine aktive Darstellung - z.B. Beugung und Streckung, die beliebig wiederholt werden kann.

Es wird eine Animation (Computer-Animation) des Gelenkmodels gesteuert durch akustische Wellen vom realen Gelenk realisiert, wobei diese Animation sowohl bei Untersuchungen, bei Therapie, bei Rehabilitation und/oder beim Training angewandt werden kann.

Es soll auch auf die Möglichkeit des diversen Anlegens einer Hilfsvorrichtung auf die ganze oder auf einen Teil der Extremität hingewiesen werden, wobei die Hilfsvorrichtung ein Gelenk oder mehrere Gelenke aufweisen kann. Außerdem soll auf die Möglichkeit der einstellbaren und meßbaren Bewegungen (und Kraftverteilung) in allen Ebenen, gekoppelt mit einstellbarer Hauptbewegung hingewiesen werden; z.B. auf die Möglichkeit zusätzlicher Distraktionen, Kompressionen und Rotationen, z.B. physiologischer Endrotationen nach außen bei Kniestreckung, zusätzlicher Kompression bei Frakturen oder nach Operationen und Nutzen der Distraktionsentlastung bzw. Wirkung auf das ganze Gelenk, nicht nur einseitiges Aufklappen oder Distraktion, analog zur Methode nach Ilisarow, z.B. bei Knochenverlängerungen.

Referenzliste

1. Proakis, John (1989), Digital Communication, McGran Hill.
2. Nyquist (1928), Certain Topics in Telegraph Transmission Theory, AIEE Trans., Vol. 47, Seiten 617-644.
3. Mac Gillem & Cooper, Continuous & Discrete Signal and System Analysis, Sanders, 1991.

4. Blahut, Fast Algorithmus for Digital Signal Processing, MA: Addison Wesley, 1985

**Patentansprüche**

1. Vorrichtung zur Ermittlung von Informationen bezüglich des anatomischen Zustands eines Bestandteils des Körpers eines Menschen oder eines Tieres, beispielsweise eines Rennpferdes oder eines Hundes, bestehend aus:

- einer Tonaufnahmevorrichtung (120, 122) zur Aufnahme von Geräuschen, die durch die Bewegung eines dem Bestandteil zugeordneten Gelenks entstehen und zur Erzeugung von entsprechenden Signalen,
- einer Analysevorrichtung zur Auswertung der Signale in Form einer Signalanalyse und
- einer Anzeigevorrichtung zur Darstellung des Ergebnisses der Signalanalyse, wobei die Analysevorrichtung durch einen Rechner (34) und die Anzeigevorrichtung durch einen dem Rechner zugeordneten Bildschirm gebildet ist, wobei eine Einrichtung oder eine Programmierung vorgesehen ist, die ausgelegt ist, um Signale zu erzeugen, die den jeweiligen Grad der Abwinklung des untersuchten Gelenkes bei der Durchführung der Bewegung angeben, und wobei der Rechner ausgelegt ist, um das Ergebnis der Signalanalyse in Form einer Animation auf dem Bildschirm darzustellen.

2. Vorrichtung nach Anspruch 1, wobei es sich bei der Analysevorrichtung entweder um eine Vorrichtung zur Durchführung einer Spektralanalyse oder um eine Vorrichtung zur Durchführung einer Analyse durch die Methoden der neuronalen Netze handelt.

3. Vorrichtung nach einem der vorhergehenden Ansprüche mit einer Hilfsvorrichtung (130, 134, 136, 140, 142, 150, 152, 156, 158, 160; 172, 174, 178, 180), die eine reproduzierbare Bewegung eines dem Bestandteil (172, 174, 178, 180) zugeordneten Gelenks ermöglicht, wobei die Hilfsvorrichtung vorzugsweise zur therapeutischen Behandlung des Gelenks ausgelegt ist und die Einstellung der Hilfsvorrichtung ebenfalls vorzugsweise anhand der aufgenommenen Tonsignale vornehmbar ist und eine Eingabevorrichtung vorzugsweise vorgesehen ist, die die Eingabe der gewollten Parameter der Bewegung der Hilfsvorrichtung gestattet.

4. Vorrichtung nach einem der vorhergehenden Ansprüche, wobei der Rechner (34) ausgelegt ist, um die Animation auf dem Bildschirm so darzustellen, dass ein auf dem Bildschirm dargestelltes Gelenk Bewegungen ausführt, die denen der jeweiligen Untersuchung entsprechen, wobei diese Bewegungen entweder synchron oder asynchron zu der eigentlichen Bewegung des Gelenks erfolgen, beispielsweise, dass zur leichteren Erfassung der vorliegenden Schäden die auf dem Bildschirm darzustellenden Bewegungen des Gelenks wesentlich langsamer darstellbar sind als die echten Bewegungen des Gelenks.

**5.** Vorrichtung nach einem der vorhergehenden Ansprüche, wobei ein Speicher oder mehrere Speicher dem Rechner (34) zugeordnet ist bzw. sind, der bzw. die zur Speicherung des Ergebnisses der Analyse ausgelegt ist bzw. sind, wobei vorzugsweise ein Speicher (202) oder mehrere Speicher, der bzw. die Referenzmuster für das Ergebnis bisheriger Signalanalysen enthält bzw. enthalten, dem Rechner (34) zugeordnet ist bzw. sind oder diesem über eine Schnittstelle zugänglich ist bzw. sind und der Rechner (34) ausgelegt ist, um für die von ihm jeweils durchgeführte Signalanalyse mindestens ein Referenzmuster abzurufen, das der Signalanalyse zumindest im wesentlichen entspricht und dieses anzuzeigen.

**6.** Vorrichtung nach Anspruch 3, wobei es sich bei der Hilfsvorrichtung um eine Schiene oder um eine Orthese (130, 134, 136, 140, 142, 150, 152, 156, 158, 160) handelt und ein Motor (150) der Hilfsvorrichtung vorzugsweise zugeordnet ist, der die Bewegung des Gelenks vorgibt, wobei der Motor (150) ebenfalls vorzugsweise über eine Schnellkupplung mit der Hilfsvorrichtung (130, 134, 136, 140, 142, 150, 152, 156, 158, 160) koppelbar und von dieser entfernbar ist und der Motor (150) gegebenenfalls vom Rechner (34) ansteuerbar ist.

**7.** Vorrichtung nach einem der vorhergehenden Ansprüche, wobei die Hilfsvorrichtung bzw. eine Hilfsvorrichtung, die eine reproduzierbare Bewegung eines dem Bestandteil zugeordneten Gelenks ermöglicht, eine Kraftmaschine (172, 174, 178, 180) ist, die zur Rehabilitation des Gelenks und/oder für das Trainieren des Gelenks bzw. der ihm zugeordneten Muskulatur ausgelegt ist.

**8.** Vorrichtung nach einem der vorhergehenden Ansprüche, wobei die Tonaufnahmeeinrichtung einen Bestandteil der Hilfsvorrichtung bzw. einer Hilfsvorrichtung, die eine reproduzierbare Bewegung eines dem Bestandteil zugeordneten Gelenks ermöglicht, bildet oder keinen Bestandteil einer solchen Hilfsvorrichtung darstellt.

**9.** Vorrichtung nach Anspruch 3, wobei es sich bei der Hilfsvorrichtung bzw. einer Hilfsvorrichtung, die eine reproduzierbare Bewegung eines dem Bestandteil zugeordneten Gelenks ermöglicht, um eine Einrichtung handelt, die in einem Sportgeschäft zur Gewinnung von Informationen über die erwünschte Einstellung eines Sportgeräts einstellbar ist.

**10.** Vorrichtung nach einem der vorhergehenden Ansprüche, **dadurch gekennzeichnet, dass** der Rechner ausgelegt ist, schadhafte Stellen durch Blinzeln oder Aufleuchten der Bildschirmdarstellung oder durch besondere Farbgebung der Bildschirmdarstellung hervorzuheben.

**11.** Vorrichtung nach Anspruch 3, wobei die Hilfsvorrichtung ausgelegt ist, um eine reproduzierbare Bewegung eines dem Bestandteil zugeordneten Gelenkes zu ermöglichen, wobei die Hilfsvorrichtung aus einer Kombination einer Orthese (130, 134, 136, 140, 142, 150, 152, 156, 158, 160) und eines Antriebsmotors (150) besteht, der für die Bewegung der Orthese und daher des Gelenkes sorgt sowie aus einem Anschluss für die Verbindung mit einem Rechner (34), der den Antriebsmotor ansteuert und/oder durchgeführte Winkelbewegungen des Gelenks erfasst, und wobei der Antriebsmotor vorzugsweise über eine Schnellkupplung mit der Hilfsvorrichtung koppelbar und von dieser entfernbar ist, damit die Orthese ohne Antriebsmotor ihre normale Funktion als Orthese erfüllen kann.

**Claims**

**1.** An apparatus for determining information with respect to the anatomic condition of a component of the body of a human or of an animal, for example of a race horse or of a dog, comprising:

- a sound recording apparatus (120, 122) for recording sounds which arise through the movement of a joint associated with the component and for producing corresponding signals,
- an analysis apparatus for evaluating the signals in the form of a signal analysis, and
- a display apparatus for illustrating the result of the signal analysis, wherein the analysis apparatus is formed by a computer (34) and the display apparatus by a screen associated with the computer; wherein a device or a program is provided which is adapted to produce signals which indicate the respective degree of angling of the investigated joint on the carrying out of the movement; and wherein the computer is designed to show the result of the signal analysis in the form of an animation on the screen.

**2.** An apparatus in accordance with claim 1, wherein the analysis apparatus is either an apparatus for the carrying out of a spectral analysis or an apparatus for the carrying out of an analysis by the methods of the neuronal networks.

3. An apparatus in accordance with any one of the preceding claims comprising an auxiliary apparatus (130, 134, 136, 140, 142, 150, 152, 156, 158, 160; 172, 174, 178, 180) enabling a reproducible movement of a joint associated with the component (172, 174, 178, 180); wherein the auxiliary apparatus is preferably designed for the therapeutic treatment of the joint and the setting of the auxiliary apparatus can likewise preferably be carried out with reference to the recorded sound signals; and wherein an input apparatus is preferably provided which allows the input of the desired parameters of the movement of the auxiliary apparatus.

4. An apparatus in accordance with any one of the preceding claims, wherein the computer (34) is designed to present the animation on the screen so that a joint shown on the screen carries out movements which correspond to those of the respective examination, with these movements taking place either synchronously or asynchronously with the actual movement of the joint, for example; wherein the movements of the joint to be presented on the screen can be shown substantially more slowly than the actual movements of the joint for the easier detection of the damage present.

5. An apparatus in accordance with any one of the preceding claims, wherein one memory or a plurality of memories is/are associated with the computer (34) which is/are adapted to store the result of the analysis; wherein preferably one memory (202) or a plurality of memories which contain/contains reference patterns for the result of previous signal analyses, is/are associated with the computer (35) or is/are accessible for the computer via an interface; and wherein the computer (34) is adapted to call up at least one reference pattern for the spectral analysis that it has carried out which corresponds at least substantially to the signal analysis and to display said reference pattern.

6. An apparatus in accordance with claim 3, wherein the auxiliary apparatus is a splint or an orthosis (130, 134, 136, 140, 142, 150, 152, 156, 158, 160); and wherein a motor (150) is preferably associated with the auxiliary apparatus and sets the movement of the joint, with the motor (150) likewise preferably being able to be coupled to the auxiliary apparatus (130, 134, 136, 140, 142, 150, 152, 155, 158, 160) via a quick coupling and being able to be disconnected therefrom, and with the motor (150) optionally being able to be controlled by the computer (34).

7. An apparatus in accordance with any one of the preceding claims, wherein the auxiliary apparatus or an auxiliary apparatus which enables a reproducible movement of a joint associated with the component is an exercise machine (172, 174, 178, 180) which is adapted for the rehabilitation of the joint and/or is adapted for the training of the joint or of the musculature associated with it.

8. An apparatus in accordance with any one of the preceding claims, wherein the sound recording device forms a component of the auxiliary apparatus or of an auxiliary apparatus which enables a reproducible movement of a joint associated with the component or does not represent a component of such an auxiliary apparatus.

9. An apparatus in accordance with claim 3, wherein the auxiliary apparatus or an auxiliary apparatus which enables a reproducible movement of a joint associated with the component is a device which can be installed in a sports shop to obtain information concerning the desired setting of an item of sport equipment.

10. An apparatus in accordance with any one of the preceding claims, wherein the computer is adapted to highlight damaged points by blinking or flashing the screen representation or by a special colouring of the screen representation.

11. An apparatus in accordance with claim 3, wherein the auxiliary apparatus is adapted to enable a reproducible movement of a joint associated with the component; wherein the auxiliary apparatus consists of a combination of an orthosis (130, 134, 136, 140, 142, 150, 152, 156, 158, 160) and of a drive motor (150) which provides the movement of the orthosis of therefore of the joint as well as of a connector for the connection to a computer (34) which controls the drive motor and/or detects the angular movements of the joint carried out; and wherein the drive motor can preferably be coupled to the auxiliary apparatus via a quick coupling and is able to be disconnected therefrom so that the orthosis can satisfy its normal function as an orthosis without a drive motor.

**Revendications**

1. Dispositif pour la détermination d'informations relatives à l'état anatomique d'un composant du corps d'un être humain ou d'un animal, par exemple d'un cheval de course ou d'un chien, comprenant :

    - un dispositif de prise de son (120, 122) pour l'enregistrement de bruits qui apparaissent en raison du mouvement d'une articulation associée au composant et pour la génération de signaux correspondants,

- un dispositif d'analyse pour exploiter les signaux sous la forme d'une analyse des signaux, et

- un dispositif d'affichage pour la représentation du résultat de l'analyse des signaux, ledit dispositif d'analyse étant formé par un calculateur (34) et le dispositif d'affichage par un écran associé au calculateur, dans lequel est prévu un système ou une programmation conçu(e) pour générer des signaux qui indiquent le degré respectif de l'angulation de l'articulation analysée lors de l'exécution du mouvement, et dans lequel le calculateur est conçu pour représenter le résultat de l'analyse des signaux sous la forme d'une animation sur l'écran.

2. Dispositif selon la revendication 1, dans lequel le dispositif d'analyse est soit un dispositif pour exécuter une analyse spectrale soit un dispositif pour exécuter une analyse par les méthodes des réseaux neuronaux.

3. Dispositif selon l'une des revendications précédentes, comprenant un dispositif auxiliaire (130, 134, 136, 140, 142, 150, 152, 156, 158, 160 ; 172, 174, 178, 180) qui permet un mouvement reproductible d'une articulation associée au composant (172, 174, 178, 180), ledit dispositif auxiliaire étant de préférence conçu pour le traitement thérapeutique de l'articulation, et le réglage du dispositif auxiliaire peut également être effectué de préférence à l'aide des signaux sonores enregistrés, et dans lequel il est de préférence prévu un dispositif de saisie qui permet la saisie des paramètres souhaités du déplacement du dispositif auxiliaire.

4. Dispositif selon l'une des revendications précédentes, dans lequel le calculateur (34) est conçu pour représenter l'animation sur l'écran de telle façon qu'une articulation illustrée sur l'écran exécute des mouvements qui correspondent à ceux de l'analyse respective, ces mouvements ayant lieu soit de façon synchrone soit de façon asynchrone par rapport au mouvement réel de l'articulation, par exemple de telle sorte que pour déterminer plus aisément les dommages existants, les mouvements de l'articulation qu'il s'agit d'illustrer sur l'écran peuvent être illustrés de façon sensiblement plus lente que les mouvements réels de l'articulation.

5. Dispositif selon l'une des revendications précédentes, dans lequel une mémoire ou plusieurs mémoires est/sont associée(s) au calculateur (34), cette mémoire/ces mémoires étant conçue(s) pour mémoriser le résultat de l'analyse, et une mémoire (202) ou plusieurs mémoires, qui contient/contiennent le modèle de référence pour le résultat d'analyse de signaux antérieurs, est/sont de préférence associée(s) au calculateur (34) et accessible(s) à celui-ci via une interface, et le calculateur (34) est conçu pour appeler, pour l'analyse des signaux exécutés respectivement par lui-même, au moins un modèle de référence qui correspond au moins sensiblement à l'analyse des signaux et pour afficher celui-ci.

6. Dispositif selon la revendication 3, dans lequel le dispositif auxiliaire est une tringle ou une orthèse (130, 134, 136, 140, 142, 150, 152, 156, 158, 160), et un moteur (150) est de préférence associé au dispositif auxiliaire, qui impose le mouvement de l'articulation, ledit moteur (150) pouvant également être accouplé au dispositif auxiliaire (130, 134, 136, 140, 142, 150, 152, 156, 158, 160), de préférence via un accouplement rapide, et pouvant être séparé de celui-ci, et le moteur (150) étant susceptible d'être piloté le cas échéant par le calculateur (34).

7. Dispositif selon l'une des revendications précédentes, dans lequel le dispositif auxiliaire, ou respectivement un dispositif auxiliaire qui permet un mouvement reproductible d'une articulation associée au composant, est une machine de force (172, 174, 178, 180) conçue pour la rééducation de l'articulation et/ou pour l'entraînement de l'articulation ou respectivement de la musculature qui lui est associée.

8. Dispositif selon l'une des revendications précédentes, dans lequel le dispositif de prise de son forme un élément du dispositif auxiliaire, ou respectivement d'un dispositif auxiliaire qui permet un mouvement reproductible d'une articulation associée au composant, ou ne représente aucun élément d'un tel dispositif auxiliaire.

9. Dispositif selon la revendication 3, dans lequel le dispositif auxiliaire, ou respectivement un dispositif auxiliaire qui permet un mouvement reproductible d'une articulation associée au composant, est un système susceptible d'être réglé dans un magasin de sport pour récupérer des informations concernant le réglage souhaité d'un appareil de sport.

10. Dispositif selon l'une des revendications précédentes, **caractérisé en ce que** le calculateur est conçu pour mettre en évidence des emplacements défectueux par clignotement ou par illumination de la représentation sur l'écran, ou par une coloration particulière de la représentation sur l'écran.

11. Dispositif selon la revendication 3, dans lequel le dispositif auxiliaire est conçu pour permettre un mouvement reproductible d'une articulation associée au composant, ledit dispositif auxiliaire étant formé par une combinaison d'une orthèse (130, 134, 136, 140, 142, 150, 152, 156, 158, 160) et d'un moteur d'entraînement (150) qui assure le mouvement de l'orthèse et donc de l'articulation, ainsi que d'un branchement pour la liaison à un ordinateur (34) qui pilote le moteur d'entraînement et/ou qui détecte les mouvements angulaires exécutés par l'articulation, et dans lequel le moteur d'entraînement est susceptible d'être couplé au dispositif auxiliaire, de préférence via un accouplement rapide, et d'être séparé de celui-ci, afin que l'orthèse puisse remplir sa fonction normale comme orthèse sans moteur d'entraînement.

# FIG. 1

EP 1 276 418 B1

# FIG. 2

digitales Signal → F F T → [38 Anzeige des Frequenzspektrums] → Ref. 1 / Ref. 2 / Ref. 3 (Vergleich mit Referenzmustern) → (+) → [38 und/oder 40 Entscheidung anzeigen bzw. ausdrucken]

30 + 32

EP 1 276 418 B1

# FIG. 3A

# FIG. 3B

## FIG. 3C

## FIG. 3D

21

## FIG. 3E

Amplitude

Zeit

10_0000_42j_gesund

## FIG. 3F

Amplitude

Zeit

6_000_64j_10%-15% op.

FIG. 4A

FIG. 4B

FIG. 4C

FIG. 4D

# FIG. 5

Comp.
Bild

34

124

122

122

120

FIG. 6A

FIG. 6B

FIG. 9A

FIG. 9B

FIG. 7B

FIG. 7A

FIG. 8B

FIG. 8A

EP 1 276 418 B1

FIG. 10

ANIMATION
NORMALES
KNIEGELENK

ANIMATION
KNIEGELENK
MIT MEDIALER
GONARTHROSE

ANIMATION
TENDOVAGINITIS
STENOSANS

ANIMATION
SCHNAPPENDE
HÜFTE

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4823807 A
- US 5213555 A
- US 5931763 A

**In der Beschreibung aufgeführte Nicht-Patentliteratur**

- **Proakis, John.** Digital Communication. McGran Hill, 1989
- **Nyquist.** Certain Topics in Telegraph Transmission Theory. *AIEE Trans.,* 1928, vol. 47, 617-644
- **Mac Gillem ; Cooper.** Continuous & Discrete Signal and System Analysis. Sanders, 1991
- **Blahut.** Fast Algorithmus for Digital Signal Processing. Addison Wesley, 1985